(19) Europäisches Patentamt
European Patent Office
Office européen des brevets

(11) Veröffentlichungsnummer: **0 063 826**
**B1**

(12) # EUROPÄISCHE PATENTSCHRIFT

(45) Veröffentlichungstag der Patentschrift: **05.12.84**

(21) Anmeldenummer: **82104991.3**

(22) Anmeldetag: **28.06.80**

(60) Veröffentlichungsnummer der früheren Anmeldung nach Art. 76 EPÜ:

(51) Int. Cl.³: **C 07 D 295/14,** C 07 C 101/54, C 07 C 101/68, C 07 D 521/00, A 61 K 31/195, A 61 K 31/395 // (C07D521/00, 209/44, 211/14, 211/46, 211/62, 211/70, 213/74, 217/04, 223/16, 265/30, 279/12, 295/20, 307/68, 491/113)

(54) **2-Amino-benzoesäure-Derivate, deren Herstellung und deren Verwendung als Arzneimittel oder Zwischenprodukte.**

(30) Priorität: **30.04.80 DE 3016650**

(43) Veröffentlichungstag der Anmeldung: **03.11.82 Patentblatt 82/44**

(45) Bekanntmachung des Hinweises auf die Patenterteilung: **05.12.84 Patentblatt 84/49**

(84) Benannte Vertragsstaaten: **AT BE CH DE FR GB IT LI LU NL SE**

(56) Entgegenhaltungen: **US - A - 3 936 467**

(73) Patentinhaber: **Dr. Karl Thomae GmbH, Postfach 1755, D-7950 Biberach (Riss) (DE)**

(72) Erfinder: **Griss, Gerhart, Dr. Dipl.-Chem., Schopperweg 1, D-7950 Biberach 1 (DE)**
Erfinder: **Sauter, Robert, Dr. Dipl.-Chem., Albert-Schweitzer-Weg 9, D-7958 Laupheim (DE)**
Erfinder: **Grell, Wolfgang, Dr. Dipl.-Chem., Amriswilstrasse 7, D-7950 Biberach 1 (DE)**
Erfinder: **Hurnaus, Rudolf, Dr. Dipl.-Chem., Silcherstrasse 19, D-7950 Biberach 1 (DE)**
Erfinder: **Eisele, Bernhard, Dr. Dipl.-Chem., Beethovenstrasse 12, D-7950 Biberach 1 (DE)**
Erfinder: **Kähling, Joachim, Dr., Haydnweg 8, D-7950 Biberach 1 (DE)**

EP 0 063 826 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

In der DE-A1-2 655 144 werden substituierte Nicotinsäureamide beschrieben, welche wertvolle Kupplungskomponenten darstellen.

Es wurde nun gefunden, dass die neuen 2-Amino-benzoesäurederivate der allgemeinen Formel I

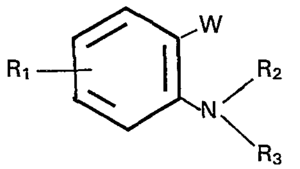

(I)

und deren Salze mit anorganischen oder organischen Säuren und auch Basen, wenn W die Carboxygruppe darstellt, wertvolle Zwischenprodukte zur Herstellung der in der EP-A1-0 023 569 beschriebenen Carbonsäurederivate darstellen, ausserdem weisen die neuen Verbindungen wertvolle pharmakologische Eigenschaften auf, nämlich eine Wirkung auf den Stoffwechsel, insbesondere jedoch eine lipidsenkende Wirkung.

Gegenstand der Erfindung sind somit neue 2-Amino-benzoesäure-Derivate der allgemeinen Formel I, deren Salze mit anorganischen oder organischen Säuren und auch Basen, wenn W die Carboxygruppe darstellt, und Verfahren zu ihrer Herstellung sowie die Verwendung der neuen Verbindungen als Zwischenprodukte oder Arzneimittel.

In der allgemeinen Formel I bedeutet

$R_1$ ein Fluor-, Chlor- oder Bromatom, eine Amino-, Cyano- oder Hydroxygruppe, eine gegebenenfalls durch eine Phenylgruppe substituierte Alkoxygruppe mit 1 bis 3 Kohlenstoffatomen oder eine Alkoxygruppe mit 4 bis 6 Kohlenstoffatomen,

$R_2$ und $R_3$ zusammen mit dem dazwischenliegenden Stickstoffatom eine N-Alkyl-cyclohexylaminogruppe, in der der Alkylteil 2 bis 4 Kohlenstoffatome enthalten kann, eine N-Alkyl-phenylamino- oder N-Alkyl-benzylaminogruppe, wobei der Alkylteil jeweils 1 bis 3 Kohlenstoffatome enthalten kann, eine cyclische Alkyleniminogruppe mit 6 bis 12 Kohlenstoffatomen, eine durch eine Alkylgruppe mit 1 bis 4 Kohlenstoffatomen, eine Alkoxygruppe mit 1 bis 3 Kohlenstoffatomen, eine Alkoxycarbonylgruppe mit insgesamt 2 bis 4 Kohlenstoffatomen oder eine Phenylgruppe substituierte Piperidinogruppe, eine durch zwei, drei oder vier Alkylgruppen mit jeweils 1 bis 3 Kohlenstoffatomen substituierte Piperidinogruppe, eine gegebenenfalls durch eine oder zwei Alkylgruppen mit jeweils 1 bis 3 Kohlenstoffatomen substituierte Morpholino- oder Thiomorpholinogruppe, eine in 4-Stellung durch eine Alkylgruppe mit 1 bis 3 Kohlenstoffatomen, eine Alkoxycarbonylgruppe mit insgesamt 2 bis 4 Kohlenstoffatomen, eine Phenyl-, Halogenphenyl-, Pyridyl-, Benzyl- oder Furoylgruppe substituierte Piperazinogruppe, eine gesättigte oder teilweise ungesättigte Aza-bicycloalkylgruppe mit 8 bis 10 Kohlenstoffatomen, eine 1,4-Dioxa-8-aza-spiro-alkangruppe mit insgesamt 6 bis 8 Kohlenstoffatomen, eine Pyrrolidino- oder Tetrahydro-pyridinogruppe und

W eine Carboxyl-, Aminocarbonyl-, Cyano- oder Carbalkoxygruppe mit insgesamt 2 bis 4 Kohlenstoffatomen.

Für die bei der Definition der Reste $R_1$, $R_2$ und $R_3$ gegebenen Bedeutungen kommt somit für

$R_1$ die Bedeutung des Fluor-, Chlor- oder Bromatoms, der Amino-, Cyano-, Hydroxy-, Methoxy-, Äthoxy-, Propoxy-, Isopropoxy-, Butoxy-, Isobutoxy-, tert.Butoxy-, Pentoxy-, Hexoxy-, Benzyloxy-, 1-Phenyläthoxy-, 2-Phenyläthoxy-, 1-Phenyl-propoxy- oder 3-Phenyl-propoxygruppe und für den Rest

$R_2$ und $R_3$ zusammen mit dem dazwischenliegenden Stickstoffatom die Bedeutung der N-Methyl-phenylamino-, N-Äthyl-phenylamino-, N-Methyl-benzylamino-, N-Äthyl-benzylamino-, N-Propyl-benzylamino-, N-Äthyl-cyclohexylamino-, N-Propyl-cyclohexylamino-, N-Isopropyl-cyclohexylamino-, N-Butyl-cyclohexylamino-, Pyrrolidino-, Hexamethylenimino-, Heptamethylenimino-, Octamethylenimino-, Nonamethylenimino-, Decamethylenimino-, Undecamethylenimino-, Dodecamethylenimino-, Methyl-piperidino-, Äthyl-piperidino-, Propyl-piperidino-, Isopropyl-piperidino-, Butyl-piperidino-, Isobutyl-piperidino-, tert.Butyl-piperidino-, Methoxy-piperidino-, Äthoxy-piperidino-, Propoxy-piperidino-, Isopropoxy-piperidino-, Methoxycarbonyl-piperidino-, Äthoxycarbonyl-piperidino-, Propoxycarbonyl-piperidino-, Isopropoxycarbonyl-piperidino-, Dimethyl-piperidino-, Trimethyl-piperidino-, Tetramethyl-piperidino-, Diäthyl-piperidino-, Dipropyl-piperidino-, Tetraäthyl-piperidino-, Methyl-äthyl-piperidino-, Äthyl-propyl-piperidino-, Morpholino-, Methyl-morpholino-, Äthyl-morpholino-, Propyl-morpholino-, Dimethyl-morpholino-, Diäthyl-morpholino-, Thiomorpholino-, Methyl-thiomorpholino-, Propyl-thiomorpholino-, Dimethyl-thiomorpholino-, N-Methyl-piperazino-, N-Äthyl-piperazino-, N-Propyl-piperazino-, N-Methoxycarbonyl-piperazino-, N-Äthoxycarbonyl-piperazino-, N-Isopropoxycarbonyl-piperazino-, N-Phenyl-piperazino-,N-Fluorphenyl-piperazino-, N-Chlorphenyl-piperazino-, N-Bromphenyl-piperazino-, N-Pyridyl-piperazino-, N-Benzyl-piperazino-, N-Furoyl-piperazino-, Octahydro-isoindol-2-yl-, Tetrahydro-isochinolin-2-yl-, Octahydro-isochinolin-2-yl-, Decahydro-isochinolin-2-yl-, Tetrahydro-3-benzazepin-3-yl-, Decahydro-3-benzazepin-3-yl-, 3-Aza-bicyclo-nonan-3-yl-, 1,4-Dioxa-8-aza-spiro[4,5]decan-8-yl-, 1,4-Dioxa-8-aza-spiro-[4,6]undecan-8-yl- oder Tetrahydropyridinogruppe in Betracht.

Bevorzugte Verbindungen der allgemeinen Formel I sind jedoch diejenigen, in denen

$R_1$ ein Chlor- oder Bromatom, eine Hydroxy-, Amino-, Cyano- oder Benzyloxygruppe oder eine Alkoxygruppe mit 1 bis 6 Kohlenstoffatomen,

$R_2$ und $R_3$ zusammen mit dem dazwischenliegenden Stickstoffatom eine N-Alkyl-cyclohexylaminogruppe, wobei der Alkylteil 2 bis 4 Kohlenstoffatome enthalten kann, eine cyclische Alkyleniminogruppe mit 6 bis 12 Kohlenstoffatomen, eine in 4-Stellung durch eine Alkylgruppe mit 1 bis 4 Kohlenstoffatomen, eine Phenyl-, Methoxy- oder Carbäthoxygruppe substituierte Piperidinogruppe, eine in 3- und 5-Stellung durch je eine Methyl- oder Äthylgruppe substituierte Piperidinogruppe, eine in 3- und 5-Stellung durch insgesamt 4 Methylgruppen substituierte

Piperidinogruppe, eine in 4-Stellung durch eine Methyl-, Benzyl-, Phenyl-, Chlorphenyl-, Pyridyl-(2)-, Carbäthoxy- oder Furoyl-(2)-gruppe substituierte Piperazinogruppe, eine gegebenenfalls durch eine oder zwei Methylgruppen substituierte Morpholino- oder Thiomorpholinogruppe, eine Pyrrolidino-, Tetrahydro-pyridino-, N-Methyl-phenylamino-, N-Methyl-benzylamino-, 1,4-Dioxa-8-aza-spiro[4,5]decan-8-yl-, 1,4-Dioxa-8-aza-spiro[4,6]undecan-8-yl-, 3-Aza-bicyclo-nonan-3-yl-, Tetrahydro-benzazepin-3-yl-, Decahydro-benzazepin-3-yl-, Tetrahydro-isochinolin-2-yl-, Octahydro-isochinolin-2-yl-, Decahydro-isochinolin-2-yl- oder Octahydro-isoindol-2-yl-Gruppe und

W die Carboxyl-, Aminocarbonyl- oder Cyangruppe bedeuten, und deren Salze.

Erfindungsgemäss erhält man die neuen Verbindungen nach folgenden Verfahren:

a) Zur Herstellung von Verbindungen der allgemeinen Formel I, in der $R_1$ eine Aminogruppe darstellt:

Reduktion einer Nitroverbindung der allgemeinen Formel II

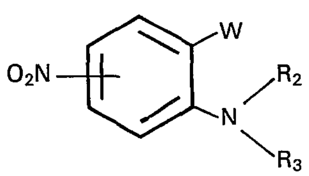

(II)

in der

$R_2$, $R_3$ und W wie eingangs definiert sind.

Die Reduktion wird zweckmässigerweise in einem Lösungsmittel wie Methanol/Äthanol, Wasser, Wasser/Äthanol, Dioxan, Methanol/Dioxan, Essigester, Dimethylformamid oder Dioxan/Dimethylformamid mit katalytisch angeregtem Wasserstoff, z.B. mit Wasserstoff in Gegenwart eines Hydrierungskatalysators wie Palladium/Kohle, Platin oder Raney-Nickel bei einem Wasserstoffdruck von 1 bis 10 bar, mit nascierendem Wasserstoff, z.B. mit Zink/Essigsäure, Zinn/Salzsäure oder Eisen/Salzsäure, oder mit einem Metallsalz, z.B. Zinn(II)-chlorid/Salzsäure oder Eisen(II)-sulfat/Schwefelsäure, bei Temperaturen zwischen 0 und 50°C, vorzugsweise jedoch bei Raumtemperatur, durchgeführt.

b) Zur Herstellung von Verbindungen der allgemeinen Formel I, inder $R_1$ eine Hydroxygruppe, eine Cyanogruppe, ein Fluor-, Chlor- oder Bromatom darstellt:

Umsetzung einer Verbindung der allgemeinen Formel Ia

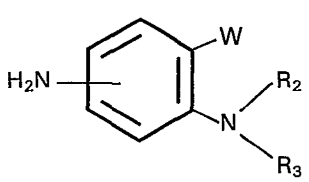

(Ia)

in der

$R_2$, $R_3$ und W wie eingangs definiert sind, mit einem Nitrit und anschliessendes Erwärmen des so erhaltenen Diazoniumsalzes gegebenenfalls in Gegenwart eines entsprechenden Kupfer(I)-Salzes.

Die Umsetzung wird zweckmässigerweise in der Weise durchgeführt, dass eine Verbindung der allgemeinen Formel Ia in einem geeigneten Lösungsmittel, z.B. in Wasser/Salzsäure, Methanol/Salzsäure oder Dioxan/Salzsäure, mit einem Nitrit, z.B. Natriumnitrit oder einem Ester der salpetrigen Säure, in ein Diazoniumsalz bei niedrigen Temperaturen, z.B. bei Temperaturen zwischen —10 und 5°C, übergeführt wird.

Das so erhaltene entsprechende Diazoniumsalz wird anschliessend z.B. als Fluoroborat, als Hydrosulfat in Schwefelsäure, als Hydrochlorid in Gegenwart von Kupfer oder in Gegenwart eines entsprechenden Kupfer(I)-Salzes wie Kupfer(I)-chlorid/Salzsäure, Kupfer(I)-bromid/Bromwasserstoffsäure oder Trinatrium-kupfer(I)-tetracyanid bei pH 7, durch Erwärmen, z.B. auf Temperaturen zwischen 15 und 90°C, in die entsprechende Verbindung übergeführt.

c) Zur Herstellung von Verbindungen der allgemeinen Formel I, in der $R_1$ eine gegebenenfalls durch eine Phenylgruppe substituierte Alkoxygruppe mit 1 bis 3 Kohlenstoffatomen oder eine Alkoxygruppe mit 1 bis 6 Kohlenstoffatomen darstellt:

Alkylierung einer Hydroxyverbindung der allgemeinen Formel Ib

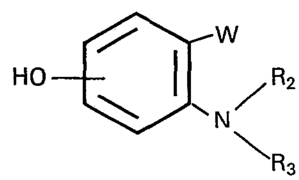

(Ib)

in der

$R_2$, $R_3$ und W wie eingangs definiert sind, mit einer Verbindung der allgemeinen Formel III

$$Z - R_1' \qquad (III)$$

in der

$R_1'$ eine gegebenenfalls durch eine Phenylgruppe substituierte Alkylgruppe mit 1 bis 3 Kohlenstoffatomen oder eine Alkylgruppe mit 4 bis 6 Kohlenstoffatomen und

Z eine nukleophile Austrittsgruppe oder zusammen mit dem benachbarten H-Atom des Restes $R_1'$ eine Diazogruppe bedeuten, und erforderlichenfalls anschliessende Hydrolyse.

Als Austrittsgruppe kommt beispielsweise ein Chlor-, Brom- oder Jodatom oder eine Sulfonyloxygruppe wie die Methansulfonyloxy-, p-Toluolsulfonyloxy- oder Methoxysulfonyloxygruppe in Betracht.

Die Umsetzung wird zweckmässigerweise in einem Lösungsmittel wie Äther, Tetrahydrofuran, Äthanol, Aceton, Dimethylformamid oder Dimethylsulfoxid gegebenenfalls in Gegenwart einer Base wie Natriumkarbonat, Kaliumkarbonat, Bariumhydroxid, Natriumäthylat oder Kalium-tert.butylat mit einem Alkylierungsmittel wie Diazomethan, Diazoäthan, Methyljodid, Äthyljodid, Isopropylbromid, Butylbromid, Dimethylsulfat, Diäthylsulfat oder p-Toluolsulfonsäuremethylester bei Temperaturen zwischen 0 und 100°C, vorzugsweise jedoch bei Temperaturen zwischen 15 und 70°C, durchgeführt.

Bedeutet hierbei W eine Carboxygruppe, so wird diese bei der Alkylierung mit einem Diazoalkan oder bei der Alkylierung in Gegenwart einer starken Base gleichzeitig verestert. Der so erhaltene Ester wird anschliessend gewünschtenfalls mittels Hydrolyse in Gegenwart einer Säure oder Base in die entsprechende Carbonsäure übergeführt.

Eine erfindungsgemäss erhaltene Verbindung der allgemeinen Formel I, in der W eine Cyano- oder Aminocarbonylgruppe darstellt, kann anschliessend mittels Hydrolyse in eine entsprechende Verbindung der allgemeinen Formel I, in der W die Carboxylgruppe darstellt, übergeführt werden.

Die nachträgliche Hydrolyse wird vorzugsweise in einem mit Wasser mischbaren Lösungsmittel wie Methanol, Äthanol, Dioxan, Wasser/Äthanol oder Wasser/Tetrahydrofuran in Gegenwart einer Säure wie Salzsäure oder Schwefelsäure oder einer Base wie Natrium- oder Kaliumhydroxid bei erhöhten Temperaturen, z.B. bei der Siedetemperatur des Reaktionsgemisches, durchgeführt.

Die so erhaltenen 2-Amino-benzoesäure-Derivate lassen sich ferner in ihre Salze mit anorganischen oder organischen Säuren oder auch Basen, wenn W die Carboxylgruppe darstellt, überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure und als Basen Natriumhydroxid oder Kaliumhydroxid in Betracht.

Die als Ausgangsstoffe verwendeten Verbindungen der allgemeinen Formeln II und II erhält man nach an sich bekannten Verfahren bzw. sind literaturbekannt. So erhält man beispielsweise eine Verbindung der allgemeinen Formel II durch Umsetzung eines entsprechenden 2-Chlor- oder 2-Brom-nitro-benzoesäure-Derivates mit einem entsprechenden Amin.

Wie bereits eingangs erwähnt, stellen die neuen Verbindungen der allgemeinen Formel I wertvolle Zwischenprodukte zur Herstellung von blutzuckersenkenden Verbindungen dar (siehe EP-A1-0 023 569) und/oder weisen wertvolle pharmakologische Eigenschaften auf, insbesondere lipidsenkende Wirkungen.

Beispielsweise wurden die Verbindungen

A = 5-Chlor-2-(1,4-dioxa-8-aza-spiro[4,6]decan-8-yl)-benzoesäure,

B = 2-(Decahydro-3-benzazepino)-5-chlor-benzoesäure,

C = 2-Decahydro-3-benzazepino)-5-brom-benzoesäure,

D = 5-Chlor-2-(4-methoxy-piperidino)-benzoesäure und

E = 5-Methoxy-2-octamethylenimino-benzoesäure

auf ihre biologischen Eigenschaften wie folgt untersucht:

1. *Lipidsenkende Wirkung*

Literatur: P. E. Schurr et al. in Atherosclerosis Drug Discovery (1976), Herausgeber: C. E. Day, Plenum, New-York, Seite 215.

Junge männliche Ratten mit einem durchschnittlichen Gewicht von 100 g wurden durch viertägige Gabe einer Diät (bestehend aus 10% Kokosfett, 1,5% Cholesterin, 0,5% Cholsäure, 0,2% Cholinchlorid und 15% Sucrose) hyperlipämisch gemacht. Unter Beibehaltung der Diät appliziert man an zwei aufeinanderfolgenden Tagen die zu untersuchenden Substanzen in Methylcellulose-Suspension per Schlundsonde. Anschliessend wurden die Tiere über Nacht nüchtern gehalten, 5 bzw. 24 Stunden nach der letzten Substanzapplikation wurde Blut zur Serumgewinnung entnommen.

Im Serum wurden Gesamtcholesterin (Boehringer Mannheim Testkombination 187.313) und Triglyceride (Boehringer Mannheim Testkombination 126.039) enzymatisch bestimmt. Die $\beta$-Lipoproteine wurden nach Fällung mit $Ca^{++}$ und Heparin im Autoanalyzer nephelometrisch bestimmt.

Die Berechnung der prozentualen Senkung erfolgte gegen eine Kontrollgruppe.

Die folgende Tabelle enthält die gefundenen Werte:

| | | Senkung in % gegenüber Kontrolle nach zweimaliger Applikation | |
|---|---|---|---|
| Substanz | Dosis [mg/kg] | Serum-Gesamtcholesterin | Serum $\beta$-Lipoproteine |
| A* | 100 | — 54 | — 54 |
| B | 100 | — 39 | — 43 |
| C | 100 | — 32 | — 32 |
| D | 100 | — 31 | — 29 |
| E* | 100 | — 29 | — 30 |

* Serumabnahme 5 Stunden nach letzter Substanzapplikation, alle anderen Werte 24 Stunden nach letzter Substanzapplikation.

2. *Akute Toxizität*

Die akute Toxizität wurde orientierend an Gruppen von 10 Mäusen (5 weibliche und 5 männliche Mäuse; Beobachtungszeit: 14 Tage) nach Gabe einer Dosis von 2000 mg/kg pro Tier in Methylcellulose-Suspension per Schlundsonde bestimmt:

| Substanz | perorale Toxizität |
|---|---|
| A | >2000 mg/kg (0 von 10 Tieren gestorben) |

Aufgrund ihrer pharmakologischen Eigenschaften eignen sich die erfindungsgemäss hergestellten Verbindungen zur Behandlung von Hyperlipidämien, insbesondere des Typs IIA, IIB und IV, und dadurch bedingten atherosklerotischen Veränderungen des Gefässsystems und lassen sich zur pharmazeutischen Anwendung, gegebenenfalls in Kombination mit anderen Wirksubstanzen, in die üblichen pharmazeutischen Zubereitungen wie Dragées, Tabletten, Kapseln, Suppositorien, Suspensionen oder Lösungen einarbeiten, die Einzeldosis beträgt hierbei 5 bis 200 mg, vorzugsweise jedoch 5 bis 50 mg, und die Tagesdosis 10 bis 500 mg, vorzugsweise 15 bis 150 mg.

Die nachfolgenden Beispiele sollen die Erfindung näher erläutern:

Herstellung der Ausgangsprodukte:

*Beispiel A*

*2-(Decahydro-isochinolin-2-yl)-5-nitro-benzoesäure*

In 500 ml Äthanol werden 19 g (0,136 Mol) Decahydro-isochinolin, 27,3 g (0,136 Mol) 2-Chlor-5-nitro-benzoesäure und 38,6 g Kaliumcarbonat unter Rühren 18 Stunden unter Rückfluss erhitzt. Nach Abdestillieren des Äthanols wird der Rückstand in 800 ml Wasser gelöst und durch Zugabe von 2N Salzsäure auf pH 4 eingestellt, wobei das Produkt auskristallisiert.

Ausbeute: 38 g (92% der Theorie),
Schmelzpunkt: 132-134°C (Isopropanol),

| | | | |
|---|---|---|---|
| Ber.: | C 63,14 | H 6,62 | N 9,20 |
| Gef.: | C 63,02 | H 6,48 | N 9,38 |

*Beispiel B*

*2-(1,4-Dioxa-8-aza-spiro[4,5]decan-8-yl)-6-nitro- -benzoesäure*

20,1 g (0,1 Mol) 2-Chlor-5-nitro-benzoesäure werden in 200 ml Äthanol mit 42,9 g (0,3 Mol) 1,4-Dioxa-8-aza-spiro[4,5]decan 8 Stunden auf Rückflusstemperatur erhitzt. Nach dem Abdestillieren des Lösungsmittels wird der Abdampfrückstand in Wasser aufgenommen und mit 2N Salzsäure auf pH 5,2 eingestellt, dabei fällt das Produkt aus. Nach Extraktion mit Chloroform und Trocknung über Natriumsulfat, kristallisiert nach dem Abdestillieren des Lösungsmittels die Verbindung aus.

Ausbeute: 12 g (39% der Theorie),
Schmelzpunkt: 155°C (Äthanol),

| | | | |
|---|---|---|---|
| Ber.: | C 54,54 | H 5,23 | N 9,09 |
| Gef.: | C 54,20 | H 5,13 | N 8,97 |

Analog den Beispielen A und B wurden folgende Verbindungen hergestellt:

2-(2-Methyl-piperidino)-5-nitro-benzoesäure
Ausbeute: 99% der Theorie, Schmelzpunkt: 164°C.
2-(3-Methyl-piperidino)-5-nitro-benzoesäure
Ausbeute: 85% der Theorie, Schmelzpunkt: 161°C.
2-(4-Methyl-piperidino)-5-nitro-benzoesäure
Ausbeute: 85% der Theorie, Schmelzpunkt: 155°C.
2-(3-Äthyl-6-methyl-piperidino)-5-nitro-benzoesäure
Ausbeute: 76% der Theorie,
Schmelzpunkt: < 20°C.
2-(3,5-Dimethyl-piperidino)-5-nitro-benzoesäure
Ausbeute: 65% der Theorie, Schmelzpunkt: 172°C.
2-(4-Methoxy-piperidino)-5-nitro-benzoesäure
Ausbeute:68% der Theorie, Schmelzpunkt: 140°C.
5-Nitro-2-(4-phenyl-piperidino)-benzoesäure
Ausbeute:88% der Theorie, Schmelzpunkt: 196°C.
2-(4-Äthoxycarbonyl-piperidino)-5-nitro-benzoesäure
Ausbeute: 82% der Theorie, Schmelzpunkt: 160°C.
5-Nitro-2-thiomorpholino-benzoesäure
Ausbeute: 80% der Theorie, Schmelzpunkt: 235°C.
5-Nitro-2-(1,2,4,5-tetrahydro-3-benzazepino)- -benzoesäure
Ausbeute: 68% der Theorie, Schmelzpunkt: 222°C.
5-Nitro-2-(1,2,3,4-tetrahydro-isochinolino)- -benzoesäure
Ausbeute: 70% der Theorie, Schmelzpunkt: 195°C.
5-Nitro-2-(4-phenyl-piperazino)-benzoesäure
Ausbeute: 88% der Theorie, Schmelzpunkt: 196°C.
5-Nitro-2-(4-pyridyl-(2)-piperazino)-benzoesäure
Ausbeute: 66% der Theorie, Schmelzpunkt: 192°C.
2-(trans-3,5-Dimethylpiperidino)-5-nitro-benzoesäure
Ausbeute: 63% der Theorie, Schmelzpunkt: 132°C.
2-(3,3,5,5-Tetramethyl-piperidino)-5-nitro- -benzoesäure
Ausbeute: 98% der Theorie, Schmelzpunkt: 138°C.
2-(3,5-Dimethyl-morpholino)-5-nitro-benzoesäure
Ausbeute: 75% der Theorie, Schmelzpunkt: 164°C.
2-(3,5-Dimethyl-thiomorpholino)-5-nitro-benzoesäure
Ausbeute: 70% der Theorie, Schmelzpunkt: 118°C.
2-(3-Aza-bicyclo[3,2,2]nonan-3-yl)-5-nitro- -benzoesäure
Ausbeute: 72% der Theorie, Schmelzpunkt: 221°C.
5-Nitro-2-nonamethylenimino-benzoesäure
Ausbeute: 80% der Theorie, Schmelzpunkt: 127°C.
5-Nitro-2-decamethylenimino-benzoesäure
Ausbeute: 92% der Theorie, Schmelzpunkt: 128°C.
5-Nitro-2-undecamethylenimino-benzoesäure
Ausbeute: 91% der Theorie, Schmelzpunkt: 120°C.
5-Nitro-2-dodecamethylenimino-benzoesäure
Ausbeute: 95% der Theorie, Schmelzpunkt: 115°C.
2-(N-Methyl-N-phenylamino)-5-nitro-benzoesäure
Ausbeute: 10% der Theorie, Schmelzpunkt: 115°C.
2-(N-Äthyl-N-cyclohexylamino-5-nitro-benzoesäure
Ausbeute: 78% der Theorie, Schmelzpunkt: 74°C.
2-(N-Butyl-N-cyclohexylamino)-5-nitro-benzoesäure
Ausbeute: 84% der Theorie, Schmelzpunkt: 56°C.
2-(N-Cyclohexyl-N-isobutylamino)-5-nitro-benzoesäure
Ausbeute: 63% der Theorie,
Schmelzpunkt: < 20°C.
2-(Decahydro-3-benzazepin-3-yl)-5-nitro-benzoesäure
Ausbeute: 98% der Theorie,
Schmelzpunkt: < 20°C.
2-(Octahydro-isoindol-2-yl)-5-nitro-benzoesäure
Ausbeute: 80% der Theorie, Schmelzpunkt: 128°C.
2-(4-Isopropyl-piperidino)-5-nitro-benzoesäure
Ausbeute: 79% der Theorie, Schmelzpunkt: 142°C.
2-(4-tert.Butyl-piperidino)-5-nitro-benzoesäure
Ausbeute: 57% der Theorie, Schmelzpunkt: 136°C.
2-(1,4-Dioxa-8-aza-spiro[4,6]undecan-8-yl)-5- -nitro-benzoesäure
Ausbeute: 75% der Theorie, Schmelzpunkt: 135°C.

2,4-Dipiperidino-5-nitro-benzoesäure
Ausbeute: 31% der Theorie, Schmelzpunkt: 152°C.

4-Chlor-2-piperidino-5-nitro-benzoesäure
Ausbeute: 18% der Theorie, Schmelzpunkt: 133°C.

5-Nitro-2-(1,2,3,6-tetrahydro-pyridino)-benzoesäure
Ausbeute: 58% der Theorie, Schmelzpunkt: 215°C.

2-(N-Methyl-N-benzylamino)-5-nitro-benzoesäure
Ausbeute: 93% der Theorie,
Schmelzpunkt: 123-126°C.

2-[4-(4-Chlorphenyl)-piperazino]-5-nitro-benzoesäure-hydrochlorid
Ausbeute: 71,5% der Theorie,
Schmelzpunkt: 225-227°C (Zers.).
2-(4-Carbäthoxy-piperazino)-5-nitro-benzoesäure
Ausbeute: 23,1% der Theorie,
Schmelzpunkt: 155-156°C.
2-[4-(2-Furoyl)-piperazino]-5-nitro-benzoesäure
Ausbeute: 64,8% der Theorie,
Schmelzpunkt: 200-205°C.
2-(4-Benzyl-piperazino)-5-nitro-benzoesäure-hydrochlorid
Ausbeute: 86,6% der Theorie,
Schmelzpunkt: 142-145°C.

*Beispiel C*

*2-Hexamethylenimino-5-nitro-benzoesäurenitril*

18,4 g (0,11 Mol) 2-Chlor-5-nitro-benzoesäurenitril werden in 250 ml Äthanol mit 22,4 g (0,21 Mol) Hexamethylenimin 4 Stunden auf Rückflusstemperatur erhitzt. Nach dem Abkühlen wird durch Zugabe von 500 ml Wasser das Produkt ölig ausgefällt. Die Fällung wird in Chloroform aufgenommen. Nach Trocknung mit Natriumsulfat und Abdestillation des Chloroforms wird der Abdampfrückstand aus Äthanol umkristallisiert.

Ausbeute: 19,7 g (73% der Theorie),
Schmelzpunkt: 70°C,

| | | | |
|---|---|---|---|
| Ber.: | C 63,65 | H 6,16 | N 17,13 |
| Gef.: | C 63,80 | H 6,07 | N 17,05 |

Herstellung der Endprodukte:

*Beispiel 1*

*5-Amino-2-(decahydro-isochinolino-2-yl)-benzoesäure*

In 250 ml Dimethylformamid werden 36 g (0,118 Mol) 2-Decahydro-isochinolin-2-yl)-5-nitro-benzoesäure gelöst und bei einem Wasserstoffdruck von 5 bar mit 10%iger Palladiumkohle als Katalysator bei Raumtemperatur hydriert. Nach beendeter Wasserstoffaufnahme wird der Katalysator abfiltriert, das Lösungsmittel im Vakuum abdestilliert und der Rückstand aus Äthanol umkristallisiert.

Ausbeute: 31,2 g (96% der Theorie),
Schmelzpunkt: 252°C,

| | | | |
|---|---|---|---|
| Ber.: | C 70,04 | H 8,08 | N 10,20 |
| Gef.: | C 70,09 | H 7,85 | N 10,12 |

*Beispiel 2*

*5-Amino-2-(1,4-dioxa-8-aza-spiro[4,5]decan-8-yl)-benzoesäure*

12 g (0,039 Mol) 2-(1,4-Dioxa-8-aza-spiro[4,5]-decan-8-yl)-5-nitro-benzoesäure werden in 100 ml Dimethylformamid bei einem Wasserstoffdruck von 5 bar bei Raumtemperatur mit 10%iger Palladiumkohle als Katalysator hydriert. Nach beendeter Wasserstoffaufnahme wird der Katalysator abfiltriert, das Lösungsmittel abdestilliert und aus Äthanol umkristallisiert.

Ausbeute: 9 g (83% der Theorie),
Schmelzpunkt: 209°C,

| | | | |
|---|---|---|---|
| Ber.: | C 60,42 | H 6,52 | N 10,07 |
| Gef.: | C 60,18 | H 6,58 | N 10,12 |

Analog den Beispielen 1 und 2 wurden folgende Verbindungen hergestellt:

5-Amino-2-pyrrolidino-benzoesäure
Ausbeute: 79% der Theorie, Schmelzpunkt: 208°C.
5-Amino-2-(2-methyl-piperidino)-benzoesäure
Ausbeute: 84% der Theorie, Schmelzpunkt: 240°C.
5-Amino-2-(3-methyl-piperidino)-benzoesäure
Ausbeute: 75% der Theorie, Schmelzpunkt: 192°C.
5-Amino-2-(4-methyl-piperidino)-benzoesäure
Ausbeute: 88% der Theorie, Schmelzpunkt: 215°C.
5-Amino-2-(3-äthyl-6-methyl-piperidino)-benzoesäure
Ausbeute: 59% der Theorie, Schmelzpunkt: 219°C.
5-Amino-2-(cis-3,5-dimethyl-piperidino)-benzoesäure
Ausbeute: 87% der Theorie, Schmelzpunkt: 234°C.
5-Amino-2-(4-methoxy-piperidino)-benzoesäure
Ausbeute: 80% der Theorie, Schmelzpunkt: 228°C.
5-Amino-2-heptamethylenimino-benzoesäure
Ausbeute: 64% der Theorie, Schmelzpunkt: 214°C.
5-Amino-2-(4-phenyl-piperidino)-benzoesäure
Ausbeute: 76% der Theorie, Schmelzpunkt: 275°C.
5-Amino-2-(4-äthoxycarbonyl-piperidino)-benzoesäure
Ausbeute: 85% der Theorie, Schmelzpunkt: 203°C.
5-Amino-2-thiomorpholino-benzoesäure
Ausbeute: 76% der Theorie, Schmelzpunkt: 193°C.
5-Amino-2-(1,2,4,5-tetrahydro-3-benzazepino)-benzoesäure
Ausbeute: 86% der Theorie, Schmelzpunkt: 258°C.
5-Amino-2-(1,2,3,4-tetrahydro-isochinolino-benzoesäure
Ausbeute: 66% der Theorie, Schmelzpunkt: 220°C.
5-Amino-2-(4-phenyl-piperazino)-benzoesäure
Ausbeute: 83% der Theorie, Schmelzpunkt: 255°C.
5-Amino-2-(4-pyridyl-(2)-piperazino)-benzoesäure
Ausbeute: 80% der Theorie, Schmelzpunkt: 248°C.
5-Amino-2-(trans-3,5-dimethyl-piperidino)-benzoesäure
Ausbeute: 89% der Theorie, Schmelzpunkt: 156°C.
5-Amino-2-(3,3,5,5-tetramethyl-piperidino)-benzoesäure
Ausbeute: 98% der Theorie,
Schmelzpunkt: < 20°C.
5-Amino-2-(3,5-dimethyl-morpholino)-benzoesäure
Ausbeute: 83% der Theorie, Schmelzpunkt: 255°C.
5-Amino-2-(3,5-dimythyl-thiomorpholino)-benzoesäure
Ausbeute: 50% der Theorie, Schmelzpunkt: 233°C.
5-Amino-2-(3-aza-bicyclo[3,2,2]nonan-3-yl)-benzoesäure
Ausbeute: 86% der Theorie, Schmelzpunkt: 288°C.
5-Amino-2-octamethylenimino-benzoesäure
Ausbeute: 88% der Theorie, Schmelzpunkt: 191°C.
5-Amino-2-nonamethylenimino-benzoesäure
Ausbeute: 80% der Theorie, Schmelzpunkt: 212°C.
5-Amino-2-decamethylenimino-benzoesäure
Ausbeute: 52% der Theorie, Schmelzpunkt: 202°C.
5-Amino-2-undecamethylenimino-benzoesäure
Ausbeute: 93% der Theorie, Schmelzpunkt: 242°C.
5-Amino-2-dodecamethylenimino-benzoesäure
Ausbeute: 59% der Theorie, Schmelzpunkt: 224°C.

5-Amino-2-(N-methyl-N-phenylamino)-benzoesäure
Ausbeute: 47% der Theorie, Schmelzpunkt: 184°C.
5-Amino-2-(N-äthyl-N-cyclohexylamino)-benzoesäure
Ausbeute: 66% der Theorie, Schmelzpunkt: 160°C.
5-Amino-2-(N-butyl-N-cyclohexylamino)-benzoesäure
Ausbeute: 48% der Theorie, Schmelzpunkt: 140°C.
5-Amino-2-(N-cyclohexyl-N-isobutylamino)-benzoesäure
Ausbeute: 62% der Theorie,
Schmelzpunkt: < 20°C.
5-Amino-2-(decahydro-3-benzazepin-3-yl)-benzoesäure
Ausbeute: 54% der Theorie, Schmelzpunkt: 204°C.
5-Amino-2-(octahydro-isoindol-2-yl)-benzoesäure
Ausbeute: 43% der Theorie, Schmelzpunkt: 228°C.
5-Amino-2-(4-isopropyl-piperidino)-benzoesäure
Ausbeute: 50% der Theorie, Schmelzpunkt: 231°C.
5-Amino-2-(4-tert.butyl-piperidino)-benzoesäure
Ausbeute: 81% der Theorie, Schmelzpunkt: 276°C.
5-Amino-2-(1,4-dioxa-8-aza-spiro[4,5]undecan-8-yl)-benzoesäure
Ausbeute: 49% der Theorie, Schmelzpunkt: 235°C.
5-Amino-2-(1,2,3,6-tetrahydro-pyridino)-benzoesäure
Ausbeute: 51% der Theorie, Schmelzpunkt: 232°C.
5-Amino-2-(4-methyl-piperazino)-benzoesäure-hydrochlorid
Ausbeute: 90% der Theorie,
Schmelzpunkt: < 20°C.
5-Amino-2-(N-methyl-N-benzylamino)-benzoesäure
Ausbeute: 95% der Theorie,
Schmelzpunkt: < 20°C.
5-Amino-2-[4-(4-chlor-phenyl)-piperazino]-benzoesäurehydrochlorid
Ausbeute: 80,5% der Theorie,
Schmelzpunkt: 305°C (Zers.).
5-Amino-2-(4-carbäthoxy-piperazino)-benzoesäure
Ausbeute: 87,5% der Theorie,
Schmelzpunkt: 195-197°C.
5-Amino-2-[4-(2-furoyl)-piperazino]-benzoesäure
Ausbeute: 97% der Theorie,
Schmelzpunkt: < 20°C.
5-Amino-2-(4-benzyl-piperazino)-benzoesäure-hydrochlorid
Ausbeute: 80% der Theorie,
Schmelzpunkt: 202-210°C.

*Beispiel 3*

*5-Chlor-2-(decahydro-isochinolin-2-yl)-benzoesäure*

In 55 ml halbkonzentrierter Salzsäure werden 10 g (0,0365 Mol) 5-Amino-2-(decahydro-isochinolin-2-yl)-benzoesäure gelöst und bei 0°C mit einer Lösung von 2,7 g (0,039 Mol) Natriumnitrit in 10 ml Wasser bei tropfenweiser Zugabe diazotiert. Nach abgeschlossener Zugabe wird 15 Minuten nachgerührt und anschliessend die Diazoniumsalzlösung in eine Suspension von 4 g Kupferpulver in 40 ml konz. Salzsäure eingetropft. Nach Rühren über Nacht entsteht eine tiefgrüne homogene Lösung, die nach Verdünnen mit 100 ml Wasser mit Chloroform erschöpfend extrahiert wird. Nach Trocknung über Natriumsulfat wird der Chloroformabdampfrückstand über eine Kieselsäule mit einem Gemisch von Essigsäureäthylester/Methanol = 9,5 : 0,5 chromatographisch gereinigt.

Ausbeute: 4,8 g (45% der Theorie),
Schmelzpunkt: 138°C,

| | | | | |
|---|---|---|---|---|
| Ber.: | C 65,41 | H 6,85 | N 4,76 | Cl 12,06 |
| Gef.: | C 65,51 | H 7,07 | N 4,89 | Cl 12,32 |

*Beispiel 4*

*5-Chlor-2-(1,4-dioxa-8-aza-spiro[4,5]decan-8-yl)-benzoesäure*

8,5 g (0,031 Mol) 5-Amino-2-(1,4-dioxa-8-aza-spiro[4,5]decan-8-yl)-benzoesäure werden in 28 ml halbkonzentrierter Salzsäure gelöst und bei 0°C mit einer Lösung von 2,4 g (0,034 Mol) Natriumnitrit in 10 ml Wasser diazotiert. Die Diazoniumsalzlösung wird unter Rühren zu einer Suspension von 3 g Kupferpulver in 3 ml konz. Salzsäure zugetropft. Nach beendeter Stickstoffentwicklung wird zwei Stunden nachgerührt, mit Wasser verdünnt und mit Chloroform ausgeschüttelt. Nach Trocknung über Natriumsulfat wird das Lösungsmittel abdestilliert. Beim Digerieren des Abdampfrückstandes mit Petroläther werden 6,1 g (66% der Ausbeute) erhalten.

Schmelzpunkt: 180°C,

| | | | |
|---|---|---|---|
| Ber.: | C 56,47 | H 5,42 | N 4,71 |
| Gef.: | C 56,11 | H 5,37 | N 4,83 |

Analog den Beispielen 3 und 4 wurden folgende Verbindungen hergestellt:

5-Chlor-2-pyrrolidino-benzoesäure
Ausbeute: 30% der Theorie, Schmelzpunkt: 164°C.
5-Chlor-2-(2-methyl-piperidino)-benzoesäure
Ausbeute: 74% der Theorie, Schmelzpunkt: 124°C.
5-Chlor-2-(3-methyl-piperidino)-benzoesäure
Ausbeute: 47% der Theorie, Schmelzpunkt: 165°C.
5-Chlor-2-(4-methylpiperidino)-benzoesäure
Ausbeute: 52% der Theorie, Schmelzpunkt: 107°C.
2-(3-Äthyl-6-methyl-piperidino)-5-chlor-benzoesäure
Ausbeute: 73% der Theorie,
Schmelzpunkt: < 20°C.
5-Chlor-2-(cis-3,5-dimethyl-piperidino)-benzoesäure
Ausbeute: 46% der Theorie, Schmelzpunkt: 167°C.
5-Chlor-2-(trans-3,5-dimethyl-piperidino)-benzoesäure
Ausbeute: 63% der Theorie, Schmelzpunkt: 132°C.
5-Chlor-2-(4-methoxy-piperidino)-benzoesäure
Ausbeute: 63% der Theorie, Schmelzpunkt: 136°C.
5-Chlor-2-heptamethylenimino-benzoesäure
Ausbeute: 58% der Theorie,
Schmelzpunkt: < 20°C.
5-Chlor-2-(4-phenyl-piperidino)-benzoesäure
Ausbeute: 51% der Theorie, Schmelzpunkt: 217°C.
5-Chlor-2-(4-äthoxycarbonyl-piperidino)-benzoesäure
Ausbeute: 97% der Theorie,
Schmelzpunkt: < 20°C.
5-Chlor-2-hexamethylenimino-benzoesäure
Ausbeute: 34% der Theorie, Schmelzpunkt: 113°C.
5-Chlor-2-thiomorpholino-benzoesäure
Ausbeute: 16% der Theorie, Schmelzpunkt: 160°C.

5-Chlor-2-(1,2,4,5-tetrahydro-3-benzazepino)-
-benzoesäure
Ausbeute: 59% der Theorie, Schmelzpunkt: 174°C.
5-Chlor-2-(1,2,3,4-tetrahydro-isochinolino)-
-benzoesäure
Ausbeute: 50% der Theorie, Schmelzpunkt: 182°C.
5-Chlor-2-(4-phenyl-piperazino)-benzoesäure
Ausbeute: 42% der Theorie, Schmelzpunkt: 154°C.
5-Chlor-2-(4-pyridyl-(2)-piperazino)-benzoesäure
Ausbeute: 45% der Theorie, Schmelzpunkt: 168°C.
5-Brom-2-(2-methyl-piperidino)-benzoesäure
Ausbeute: 31% der Theorie, Schmelzpunkt: 168°C.
5-Chlor-2-(3,3,5,5-tetramethyl-piperidino)-benzoesäure
Ausbeute: 62% der Theorie,
Schmelzpunkt: < 20°C.
5-Brom-2-(4-methoxy-piperidino)-benzoesäure
Ausbeute: 48% der Theorie, Schmelzpunkt: 138°C.
5-Chlor-2-(3,5-dimethyl-morpholino)-benzoesäure
Ausbeute: 50% der Theorie, Schmelzpunkt: 174°C.
5-Chlor-2-(3,5-dimethyl-thiomorpholino)-benzoesäure
Ausbeute: 18% der Theorie, Schmelzpunkt: 134°C.
5-Brom-2-heptamethylenimino-benzoesäure
Ausbeute: 15% der Theorie, Schmelzpunkt: 104°C.
5-Chlor-2-(3-aza-bicyclo[3,2,2]nonan-3-yl)-
-benzoesäure
Ausbeute: 16% der Theorie, Schmelzpunkt: 199°C.
5-Chlor-2-octamethylenimino-benzoesäure
Ausbeute: 70% der Theorie, Schmelzpunkt: 84°C.
5-Chlor-2-nonamethylenimino-benzoesäure
Ausbeute: 30% der Theorie, Schmelzpunkt: 78°C.
5-Chlor-2-decamethylenimino-benzoesäure
Ausbeute: 65% der Theorie, Schmelzpunkt: 70°C.
5-Chlor-2-undecamethylenimino-benzoesäure
Ausbeute: 41% der Theorie, Schmelzpunkt: 41°C.
5-Chlor-2-dodecamethylenimino-benzoesäure
Ausbeute: 36% der Theorie, Schmelzpunkt: 40°C.
5-Chlor-2-(N-phenyl-N-methylamino)-benzoesäure
Ausbeute: 27% der Theorie, Schmelzpunkt: 164°C.
2-(N-Äthyl-N-cyclohexylamino)-5-chlor-benzoesäure
Ausbeute: 24% der Theorie, Schmelzpunkt: 152°C.
2-(N-Butyl-N-cyclohexylamino)-5-chlor-benzoesäure
Ausbeute: 16% der Theorie, Schmelzpunkt: 145°C.
5-Chlor-2-(N-cyclohexyl-N-isobutylamino)-benzoesäure
Ausbeute: 22% der Theorie, Schmelzpunkt: 131°C.
5-Chlor-2-(decahydro-3-benzazepin-3-yl)-benzoesäure
Ausbeute: 70% der Theorie, Schmelzpunkt: 153°C.
5-Brom-2-(decahydro-3-benzazepin-3-yl)-benzoesäure
Ausbeute: 54% der Theorie, Schmelzpunkt: 154°C.

5-Chlor-2-(octahydro-isoindol-2-yl)-benzoesäure
Ausbeute: 33% der Theorie, Schmelzpunkt: 164°C.

5-Brom-2-octamethylenimino-benzoesäure
Ausbeute: 48% der Theorie, Schmelzpunkt: 94°C.

5-Chlor-2-(4-isopropyl-piperidino)-benzoesäure
Ausbeute: 43% der Theorie, Schmelzpunkt: 172°C.

5-Chlor-2-(-4-tert.butyl-piperidino)-benzoesäure
Ausbeute: 35% der Theorie, Schmelzpunkt: 161°C.

5-Chlor-2-(1,4-dioxa-8-aza-spiro[4,6]undecan-8-
-yl)-benzoesäure
Ausbeute: 42% der Theorie, Schmelzpunkt: 163°C.
5-Chlor-2-(1,2,3,6-tetrahydro-pyridino)-benzoesäure
Ausbeute: 73% der Theorie, Schmelzpunkt: 173°C.
5-Chlor-2-(4-methyl-piperazino)-benzoesäure-
-hydrochlorid
Ausbeute: 75% der Theorie,
Schmelzpunkt: 132°C (Zers.).
5-Chlor-2-(N-methyl-N-benzylamino)-benzoesäure
Ausbeute: 18,2% der Theorie,
Schmelzpunkt: 156-157°C.
5-Chlor-2-[4-(4-chlor-phenyl)-piperazino]benzoesäure
Ausbeute: 30,5% der Theorie,
Schmelzpunkt: 228-230°C.
2-(4-Carbäthoxy-piperazino)-5-chlor-benzoesäure
Ausbeute: 52% der Theorie,
Schmelzpunkt: 129-130°C.
5-Chlor-2-[4-(2-furoyl)-piperazino]benzoesäure
Ausbeute: 33,1% der Theorie,
Schmelzpunkt: 200-202°C.
2-(4-Benzyl-piperazino)-5-chlor-benzoesäure-
-hydrochlorid
Ausbeute: 42,8% der Theorie,
Schmelzpunkt: 2230-232°C (Zers.).

*Beispiel 5*

*5-Amino-2-hexamethylenimino-benzoesäurenitril*

21,4 g (0,087 Mol) 2-Hexamethylenimino-5-nitro-benzoesäurenitril werden in 200 ml Dioxan und 500 ml Methanol gelöst und bei Raumtemperatur und einem Wasserstoffdruck von 5 bar in Gegenwart von 10%iger Palladiumkohle als Katalysator hydriert. Nach Abfiltrieren des Katalysators und Abdestillieren des Lösungsmittels werden 20 g (100% der Theorie) erhalten.

Schmelzpunkt: < 20°C.

*Beispiel 6*

*5-Chlor-2-hexamethylenimino-benzoesäurenitril*

20 g (0,092 Mol) 5-Amino-2-hexamethylenimino-benzoesäurenitril werden in 90 ml halbkonzentrierter Salzsäure gelöst und bei 0°C mit einer Lösung von 6,5 g (0,094 Mol) Natriumnitrit in 30 ml Wasser bei tropfenweiser Zugabe diazotiert. Nach beendeter Zugabe wird 15 Minuten nachgerührt. Die Diazoniumsalzlösung wird unter Rühren zu einer Lösung von Kupfer(I)-chlorid in konz. Salzsäure, die auf 70°C erwärmt wird, zugetropft. Nach abgeschlossener Stickstoffentwicklung wird mit Chloroform extrahiert. Nach Trocknung über Natriumsulfat und Abdestillieren des Chloroforms wird der Abdampfrückstand über eine Kieselgelsäule chromatographisch gereinigt. Als Fliessmittel wird Toluol verwendet.

Ausbeute: 5 g (23% der Theorie),
Schmelzpunkt: < 20°C.

*Beispiel 7*

*5-Chlor-2-hexamethylenimino-benzoesäure*

5 g (0,021 Mol) 5-Chlor-2-hexamethylenimino--benzoesäurenitril werden in 32 g Kalilauge und 20 ml Wasser 8 Stunden auf 170°C erhitzt. Die erkaltene Schmelze wird in Wasser gelöst. Durch Ansäuern auf pH 5 wird das Amid quantitativ ausgefällt, welches anschliessend mit halbkonzentrierter Salzsäure hydrolysiert wird.

Ausbeute: 3,6 g (67,4% der Theorie),
Schmelzpunkt: 113°C.

Analog den Beispielen 5 bis 7 wurden folgende Verbindungen hergestellt:

2-Morpholino-5-nitro-benzoesäurenitril
Ausbeute: 78% der Theorie, Schmelzpunkt: 138°C.
5-Amino-2-morpholino-benzoesäurenitril
Ausbeute: 68% der Theorie, Schmelzpunkt: 142°C.
5-Chlor-2-morpholino-benzoesäurenitril
Ausbeute: 20% der Theorie, Schmelzpunkt: 57°C.
5-Chlor-2-morpholino-benzamid
Ausbeute: 98% der Theorie, Schmelzpunkt: 280°C.
5-Chlor-2-morpholino-benzoesäure
Ausbeute: 60% der Theorie, Schmelzpunkt: 157°C.

*Beispiel 8*

*5-Cyano-2-octamethylenimino-benzoesäure*

26,2 g (0,1 Mol) 5-Amino-2-octamethylenimino-benzoesäure werden in 38 ml konz. Salzsäure gelöst, mit 280 ml Wasser verdünnt und bei 0°C mit einer Lösung von 7,6 g (0,11 Mol) Natriumnitrit in 30 ml Wasser bei tropfenweiser Zugabe diazotiert. Nach halbstündigem Nachrühren wird die Lösung mit Natriumkarbonat auf pH 7 gestellt. Zu der Diazoniumsalzlösung wird anschliessend eine Lösung von Trinatrium-tetracyano-kupfer(I)-Komplex bei 0°C zugetropft.

Diese Kupfer(I)-Komplex-Lösung wird wie folgt erhalten: 32 g (0,128 Mol) Kupfersulfat × 5 $H_2O$ und 8,7 g Natriumchlorid in 100 ml Wasser werden mit einer Natriumhydrogensulfitlösung, bestehend aus 6,6 g (0,0635 Mol) Natriumhydrogensulfit, 4,4 g Natriumhydroxid in 50 ml Wasser zum Kupfer(I)-chlorid reduziert. Das ausgefallene Kupfer(I)-chlorid wird abgesaugt, in 50 ml Wasser suspendiert und in einer Lösung von 17 g (0,346 Mol) Natriumcyanid in 30 ml Wasser gelöst.

Nach abgeschlossener Stickstoffentwicklung wird das Reaktionsgemisch eine Stunde auf 70°C erwärmt. Nach dem Erkalten wird mit 2N Salzsäure pH 5,5 eingestellt und mit Chloroform extrahiert. Die Chloroformphasen werden über Natriumsulfat getrocknet und nach Abdestillieren des Chloroforms wird das erhaltene Rohprodukt über eine Kieselgelsäule mit Essigsäureäthylester als Fliessmittel gereinigt.

Ausbeute: 9 g (30% der Theorie),
Schmelzpunkt: < 20°C,

| | | | |
|---|---|---|---|
| Ber.: | C 70,56 | H 7,40 | N 10,28 |
| Gef.: | C 70,38 | H 7,20 | N 10,10 |

*Beispiel 9*

*2-Heptamethylenimino-5-hydroxy-benzoesäure*

26,7 g (0,107 Mol) 5-Amino-2-heptamethylenimino-benzoesäure werden in 190 ml 3N Schwefelsäure gelöst und bei 0°C mit einer Lösung von 8,3 g (0,12 Mol) Natriumnitrit in 30 ml Wasser bei tropfenweiser Zugabe diazotiert. Nach halbstündigem Nachrühren werden 2 g feingepulverter Harnstoff zugesetzt. Bei guter Rührung wird die Diazoniumsalzlösung zu 320 ml 50%iger Schwefelsäure, die auf 90°C erwärmt ist, zugetropft. Nach beendeter Stickstoffentwicklung wird bei Raumtemperatur mit Ammoniak auf pH 4 eingestellt und mit Chloroform extrahiert. Der nach Trocknung über Natriumsulfat und Abdestillieren des Chloroforms gewonnene Trockenrückstand wird über eine Kieselgelsäule mit Chloroform als Laufmittel gereinigt.

Nach Umkristallisieren aus Isopropanol werden 8 g (30% der Theorie) erhalten.

Schmelzpunkt: 199°C,

| | | | |
|---|---|---|---|
| Ber.: | C 67,45 | H 7,68 | N 5,61 |
| Gef.: | C 66,87 | H 7,71 | N 6,65 |

Analog Beispiel 9 wurden folgende Verbindungen hergestellt:

2-Hexamethylenimino-5-hydroxy-benzoesäure
Ausbeute: 24% der Theorie, Schmelzpunkt: 214°C.
5-Hydroxy-2-octamethylenimino-benzoesäure
Ausbeute: 27% der Theorie, Schmelzpunkt: 208°C.
5-Hydroxy-2-(4-tert.butyl-piperidino)-benzoesäure
Ausbeute: 33% der Theorie, Schmelzpunkt: 240°C.
5-Hydroxy-2-(cis-3,5-dimethyl-piperidino)-benzoesäure
Ausbeute: 67,4% der Theorie,
Schmelzpunkt: 248-250°C.

*Beispiel 10*

*5-Methoxy-2-octamethylenimino-benzoesäure*

3,2 g (12,2 Mol) 5-Hydroxy-2-octamethylenimino-benzoesäure werden in 20 ml absolutem Dimethylformamid mit 0,6 g (25 mMol) Natriumhydroxid versetzt und auf 50°C erwärmt, dabei fällt zum Teil das Natriumsalz aus. Nach Zugabe von 5,2 g (36,6 mMol) Methyljodid in 3 ml absolutem Dimethylformamid wird 5 Stunden bei Raumtemperatur gerührt. Nach Abdestillieren des Dimethylformamids wird der rohe 5-Methoxy-2-octamethylenimino-benzoesäure-methylester über eine Kieselgelsäule mit Chloroform als Laufmittel gereinigt.

Ausbeute: 90% der Theorie,
Schmelzpunkt: < 20°C.

Dieser Ester wird mit Natronlauge bei 80°C hydrolisiert. Nach dem Ansäuern auf pH 5,2 wird mit Chloroform extrahiert, über Natriumsulfat getrocknet und der Abdampfrückstand mit Petroläther digeriert.

Ausbeute: 85% der Theorie,
Schmelzpunkt: 84°C,

| | | | |
|---|---|---|---|
| Ber.: | C 69,28 | H 8,35 | N 5,04 |
| Gef.: | C 69,12 | H 8,29 | N 4,95 |

Analog Beispiel 10 wurden folgende Verbindungen hergestellt:

2-Hexamethylenimino-5-methoxy-benzoesäure
Ausbeute: 88% der Theorie, Schmelzpunkt: 141°C.
2-Heptamethylenimino-5-methoxy-benzoesäure
Ausbeute: 30% der Theorie, Schmelzpunkt: 120°C.
2-Heptamethylenimino-5-isopropyloxy-benzoesäure
Ausbeute: 78% der Theorie, Schmelzpunkt: 120°C.

5-Äthoxy-2-octamethylenimino-benzoesäure
Ausbeute: 87% der Theorie,
Schmelzpunkt: < 20°C.
5-Isopropyloxy-2-octamethylenimino-benzoesäure
Ausbeute: 60% der Theorie, Schmelzpunkt: 78°C.
5-Butyl-(2)-oxy-2-octamethylenimino-benzoesäure
Ausbeute: 48% der Theorie,
Schmelzpunkt: < 20°C.
5-Methoxy-3-(4-tert.butyl-piperidino)-benzoesäure
Ausbeute: 22% der Theorie, Schmelzpunkt: 156°C.
5-Methoxy-2-(3,5-cis-dimethyl-piperidino)-benzoesäure
Ausbeute: 90% der Theorie, Schmelzpunkt: 124°C.
5-Hexyloxy-2-piperidino-benzoesäure
Ausbeute: 73% der Theorie, Schmelzpunkt: 72°C.
5-Benzyloxy-2-piperidino-benzoesäure
Ausbeute: 41% der Theorie, Schmelzpunkt: 188°C.

*Beispiel I*

*Suppositorien zu 30 mg 5-Chlor-2-(1,4-dioxa-8-aza--spiro[4,6]decan-8-yl)-benzoesäure*

Zusammensetzung:

| | |
|---|---|
| 1 Zäpfchen enthält | |
| Wirksubstanz | 0,030 g |
| Zäpfchenmasse (z.B. Witepsol W 45 | 1,336 g |
| Witepsol E 75 | 0,334 g |
| | 1,700 g |

*Herstellung:*

Der gemahlene Wirkstoff wird unter Rühren in das auf 40°C temperierte aufgeschmolzene Gemisch der Zäpfchenmasse eingetragen und die Schmelze in gekühlte Giessformen ausgegossen. Nach dem völligen Erstarren werden die Suppositorien den Formen entnommen und in geeigneter Weise verpackt.

*Beispiel II*

*Gelatine-Steckkapseln zu 5 mg 5-Chlor-2-(1,4--dioxa-8-aza-spiro[4,6]decan-8-yl)-benzoesäure*

| | |
|---|---|
| 1 Kapsel enthält: | |
| Wirksubstanz | 5,0 mg |
| Maisstärke getr. | 100,0 mg |
| Maisstärke pulv. | 93,0 mg |
| Magnesiumstearat | 2,0 mg |
| | 200,0 mg |

*Herstellung:*

Wirkstoff und Hilfsstoffe werden gemischt, durch ein Sieb der Maschenweite 0,75 mm gegeben und in einem geeignetem Mischer homogen verteilt. Das Pulver wird auf einem Kapselfüll- und Schliessautomaten in Hartgelatine-Steckkapseln der Grösse 3 (Parke Davis) abgefüllt.

*Beispiel III*

*Tabletten zu 25 mg 5-Chlor-2-(1,4-dioxa-8-aza--spiro[4,6]decan-8-yl)-benzoesäure*

| | |
|---|---|
| 1 Tablette enthält: | |
| Wirksubstanz | 25,0 mg |
| Milchzucker | 35,0 mg |
| Maisstärke | 15,0 mg |
| Polyvinylpyrrolidon | 4,5 mg |
| Magnesiumstearat | 0,5 mg |
| | 80,0 mg |

*Herstellung:*

Der Wirkstoff wird mit Milchzucker und Stärke gemischt und danach mit der wässrigen Polyvinylpyrrolidon-Lösung gleichmässig befeuchtet.

| | |
|---|---|
| Feuchtsiebung: | 1,5-mm-Maschenweite |
| Trocknung: | Umlufttrockenschrank bei 45°C |
| Trockensiebung: | 1,0-mm-Maschenweite |

Das trockne Granulat wird nach Zumischung des Schmiermittels zu Tabletten verpresst.
Tabletten: 6 mm Ø, beidseitige Facette, einseitige Teilkerbe, biplan.

*Beispiel IV*

*Dragées zu 25 mg 5-Chlor-2-(1,4-dioxa-8-aza--spiro[4,6]decan-8-yl)-benzoesäure*

Herstellung der pressfertigen Mischung analog Beispiel II.
Verpressung zu bikonvexen Drageekernen von 80,0 mg Gewicht, 6 mm Ø und Wölbungsradius 5 mm.
Die Kerne werden mit einer praxisüblichen Zuckerdragiersuspension auf ein Gewicht von 110 mg im Dragierkessel dragiert und anschliessend mit einer Poliersuspension poliert.

**Patentansprüche für die Vertragsstaaten:**
BE, CH, DE, FR, GB, IT, LI, LU, NL,SE

1. 2-Amino-benzoesäure-Derivate der allgemeinen Formel I

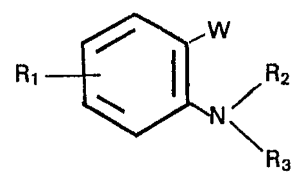

(I)

in der
$R_1$ ein Fluor-, Chlor- oder Bromatom, eine Amino-, Cyano- oder Hydroxygruppe, eine gegebenenfalls durch eine Phenylgruppe substituierte Alkoxygruppe mit 1 bis 3 Kohlenstoffatomen oder eine Alkoxygruppe mit 4 bis 6 Kohlenstoffatomen,
$R_2$ und $R_3$ zusammen mit dem dazwischenliegenden Stickstoffatom eine N-Alkyl-cyclohexylaminogruppe, in der der Alkylteil 2 bis 4 Kohlenstoffatome enthalten kann, eine N-Alkyl-phenylamino- oder N-Alkyl-benzylaminogruppe, wobei der Alkylteil jeweils 1 bis 3 Kohlenstoffatome enthalten kann, eine cyclische Alkyleniminogruppe mit 6 bis 12 Kohlenstoffatomen, eine durch eine Alkylgruppe mit 1 bis 4 Kohlenstoffatomen, eine Alkoxygruppe mit 1 bis 3 Kohlenstoffatomen, eine Alkoxycarbonylgruppe mit insgesamt 2 bis 4 Kohlenstoffatomen oder eine Phenylgruppe substituierte Piperidinogruppe, eine durch 2, 3 oder 4 Alkylgruppen mit jeweils 1 bis 3 Kohlenstoffatomen substituierte Piperidinogruppe, eine gegebenenfalls durch eine oder zwei Alkylgruppen mit jeweils 1 bis 3 Kohlenstoffatomen substituierte Morpholino- oder Thiomorpholinogruppe, eine in 4-Stellung durch eine Alkylgruppe mit 1 bis 3 Kohlenstoffatomen, eine Alkoxycarbonylgruppe mit insgesamt 2 bis 4 Kohlenstoffatomen, eine Phenyl-, Halogenphenyl-, Pyridyl-, Benzyl- oder Furoylgruppe substituierte Piperazinogruppe, eine gesättigte oder teilweise ungesättigte Aza-bicycloalkylgruppe mit 8 bis 10 Kohlenstoffatomen, eine 1,4-Dioxa-8-aza-spiro-alkangruppe mit insgesamt 6 bis 8 Kohlenstoffatomen, eine Pyrrolidino- oder Tetrahydro-pyridinogruppe und

W eine Carboxyl-, Aminocarbonyl-, Cyano- oder Carbalkoxygruppe mit insgesamt 2 bis 4 Kohlenstoffatomen bedeuten, und deren Säureadditionssalze mit anorganischen oder organischen Säuren oder auch Basen, wenn W die Carboxylgruppe darstellt.

2. 2-Amino-benzoesäure-Derivate der allgemeinen Formel I gemäss Anspruch 1, in der

$R_1$ ein Chlor- oder Bromatom, eine Hydroxy-, Amino-, Cyano- oder Benzyloxygruppe oder eine Alkoxygruppe mit 1 bis 6 Kohlenstoffatomen,

$R_2$ und $R_3$ zusammen mit dem dazwischenliegenden Stickstoffatom eine N-Alkyl-cyclohexylaminogruppe, wobei der Alkylteil 2 bis 4 Kohlenstoffatome enthalten kann, eine cyclische Alkyleniminogruppe mit 6 bis 12 Kohlenstoffatomen, eine in 4-Stellung durch eine Alkylgruppe mit 1 bis 4 Kohlenstoffatomen, eine Phenyl-, Methoxy- oder Carbäthoxygruppe substituierte Piperidinogruppe, eine in 3- und 5-Stellung durch je eine Methyl- oder Äthylgruppe substituierte Piperidinogruppe, eine in 3- und 5-Stellung durch insgesamt 4 Methylgruppen substituierte Piperidinogruppe, eine in 4-Stellung durch eine Methyl-, Benzyl-, Phenyl-, Chlorphenyl-, Pyridyl-(2)-, Carbäthoxy- oder Furoyl-(2)-gruppe substituierte Piperazinogruppe, eine gegebenenfalls durch eine oder zwei Methylgruppen substituierte Morpholino- oder Thiomorpholinogruppe, eine Pyrrolidino-, Tetrahydro-pyridino-, N-Methyl-phenylamino-, N-Methyl-benzylammino-, 1,4-Dioxa-8-aza-spiro[4,5]decan-8-yl-, 1,4-Dioxa-8-aza-spiro[4,6]undecan-8-yl-, 3-Aza-bicyclo-nonan-3-yl-, Tetrahydro-benzazepin-3-yl-, Decahydro-benzazepin-3-yl-, Tetrahydro-isochinolin-2-yl-, Octahydro-isochinolin-2-yl-, Decahydro-isochinolin-2-yl- oder Octahydro-isoindol-2-yl-Gruppe und

W die Carboxy-, Aminocarbonyl- oder Cyangruppe bedeuten, und deren Säureadditionssalze mit anorganischen oder organischen Säuren oder auch Basen, wenn W die Carboxylgruppe darstellt.

3. 2-Amino-benzoesäure-Derivate der allgemeinen Formel I gemäss Anspruch 1, in der

$R_1$ eine Alkoxygruppe mit 1 bis 3 Kohlenstoffatomen, ein Chlor- oder Bromatom,

$R_2$ und $R_3$ mit dem dazwischenliegenden Stickstoffatom eine cyclische Alkyleniminogruppe mit 6 bis 8 Kohlenstoffatomen, eine 1,4-Dioxa-8-aza-spiro[4,5]decan-8-yl-, 1,4-Dioxa-8-aza-spiro[4,6]undecan-8-yl, Decahydro-3-benzazepino- oder 4-Methoxy-piperidinogruppe und

W die Carboxylgruppe bedeuten, und deren Säureadditionssalze mit anorganischen oder organischen Säuren oder Basen.

4. 2-Amino-benzoesäure-Derivate der allgemeinen Formel I gemäss Anspruch 1, in der $R_1$ in 5-Position steht und $R_1$, $R_2$, $R_3$ und W wie im Anspruch 3 definiert sind, und deren Säureadditionssalze mit anorganischen oder organischen Säuren oder Basen.

5. 5-Chlor-(1,4-dioxa-8-aza-spiro[4,6]decan-8-yl)-benzoesäure und dessen Säureadditionssalze.

6. Arzneimittel, enthaltend eine Verbindung gemäss den Ansprüchen 1 bis 5 neben einem oder mehreren inerten Trägerstoffen und/oder Verdünnungsmitteln.

7. Arzneimittel gemäss Anspruch 6 mit lipidsenkenden Eigenschafften.

8. Verfahren zur Herstellung von 2-Amino-benzoesäure-Derivaten der allgemeinen Formel I gemäss den Ansprüchen 1 bis 5, sowie von deren Säureadditionssalzen mit anorganischen oder organischen Säuren oder auch Basen, wenn W die Carboxylgruppe darstellt, dadurch gekennzeichnet, dass

a) zur Herstellung von Verbindungen der allgemeinen Formel I, in der $R_1$ eine Aminogruppe darstellt, eine Nitroverbindung der allgemeinen Formel II

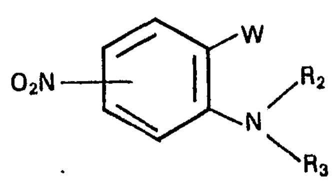

(II)

in der

$R_2$, $R_3$ und W wie eingangs definiert sind, reduziert wird oder

b) zur Herstellung von Verbindungen der allgemeinen Formel I, in der $R_1$ eine Hydroxygruppe, eine Cyanogruppe, ein Fluor-, Chlor- oder Bromatom darstellt, eine Verbindung der allgemeinen Formel Ia

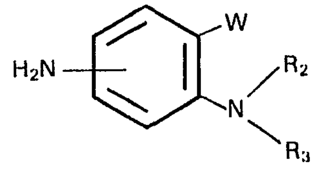

(Ia)

in der

$R_2$, $R_3$ und W wie eingangs definiert sind, mit einem Nitrit in ein Diazoniumsalz übergeführt wird und das so erhaltene Diazoniumsalz anschliessend in Gegenwart von Schwefelsäure, in Gegenwart von Kupfer oder in Gegenwart eines entsprechenden Kupfer(I)-Salzes durch Erwärmen in die entsprechende Verbindung übergeführt wird oder

c) zur Herstellung von Verbindungen der allgemeinen Formel I, in der $R_1$ eine gegebenenfalls durch eine Phenylgruppe substituierte Alkoxygruppe mit 1 bis 3 Kohlenstoffatomen oder eine Alkoxygruppe mit

4 bis 6 Kohlenstoffatomen darstellt, eine Hydroxyverbindung der allgemeinen Formel Ib

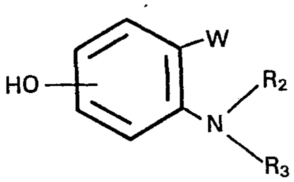

(Ib)

in der

$R_2$, $R_3$ und W wie eingangs definiert sind, mit einer Verbindung der allgemeinen Formel III

$$Z - R_1' \qquad (III)$$

in der

$R_1'$ eine gegebenenfalls durch eine Phenylgruppe substituierte Alkylgruppe mit 1 bis 3 Kohlenstoffatomen oder eine Alkylgruppe mit 4 bis 6 Kohlenstoffatomen

und Z eine nukleophile Austrittsgruppe oder zusammen mit dem benachbarten Wasserstoffatom des Restes $R_1'$ eine Diazogruppe bedeuten, alkyliert und erforderlichenfalls anschliessend hydrolisiert wird und gewünschtenfalls anschliessend eine so erhaltene Verbindung der allgemeinen Formel I, in der W eine Cyano-, Aminocarbonyl- oder Alkoxycarbonylgruppe darstellt, mittels Hydrolyse in eine entsprechende Verbindung der allgemeinen Formel I, in der W die Carboxylgruppe darstellt, übergeführt wird und/oder eine so erhaltene Verbindung der allgemeinen Formel I in ihre Säureadditionssalze mit anorganischen oder organischen Säuren oder auch Basen, wenn W die Carboxylgruppe darstellt, übergeführt wird.

9. Verfahren gemäss Anspruch 8, dadurch gekennzeichnet, dass die Umsetzung in einem Lösungsmittel durchgeführt wird.

10. Verfahren gemäss den Ansprüchen 8b und 9, dadurch gekennzeichnet, dass das so hergestellte Diazoniumsalz, z.B. das Fluoroborat, das Hydrosulfat in Gegenwart von Schwefelsäure oder in Gegenwart eines entsprechenden Kupfer(I)-Salzes wie Kupfer(I)-chlorid/Salzsäure, Kupfer(I)-bromid/Bromwasserstoffsäure oder Trinatrium-kupfer(I)-tetracyanid bei pH 7, durch Erwärmen in die entsprechende Verbindung übergeführt wird.

**Patentansprüche für den Vertragsstaat:** AT

1. Verfahren zur Herstellung von 2-Amino-benzoesäure-Derivaten der allgemeinen Formel I

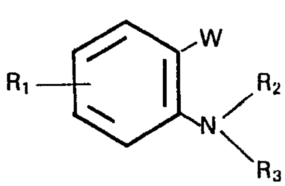

(I)

in der

$R_1$ ein Fluor-, Chlor- oder Bromatom, eine Amino-, Cyano- oder Hydroxygruppe, eine gegebenenfalls durch eine Phenylgruppe substituierte Alkoxygruppe mit 1 bis 3 Kohlenstoffatomen oder eine Alkoxygruppe mit 4 bis 6 Kohlenstoffatomen,

$R_2$ und $R_3$ zusammen mit dem dazwischenliegenden Stickstoffatom eine N-Alkyl-cyclohexylaminogruppe, in der der Alkylteil 2 bis 4 Kohlenstoffatome enthalten kann, eine N-Alkyl-phenylamino- oder N-Alkyl-benzylaminogruppe, wobei der Alkylteil jeweils 1 bis 3 Kohlenstoffatome enthalten kann, eine cyclische Alkyleniminogruppe mit 6 bis 12 Kohlenstoffatomen, eine durch eine Alkylgruppe mit 1 bis 4 Kohlenstoffatomen, eine Alkoxygruppe mit 1 bis 3 Kohlenstoffatomen, eine Alkoxycarbonylgruppe mit insgesamt 2 bis 4 Kohlenstoffatomen oder eine Phenylgruppe substituierte Piperidinogruppe, eine durch zwei, drei oder vier Alkylgruppen mit jeweils 1 bis 3 Kohlenstoffatomen substituierte Piperidinogruppe, eine gegebenenfalls durch eine oder zwei Alkylgruppen mit jeweils 1 bis 3 Kohlenstoffatomen substituierte Morpholino- oder Thiomorpholinogruppe, eine in 4-Stellung durch eine Alkylgruppe mit 1 bis 3 Kohlenstoffatomen, eine Alkoxycarbonylgruppe mit insgesamt 2 bis 4 Kohlenstoffatomen, eine Phenyl-, Halogenphenyl-, Pyridyl-, Benzyl- oder Furoylgruppe substituierte Piperazinogruppe, eine gesättigte oder teilweise ungesättigte Aza-bicycloalkylgruppe mit 8 bis 10 Kohlenstoffatomen, eine 1,4-Dioxa-8-aza-spiro-alkangruppe mit insgesamt 6 bis 8 Kohlenstoffatomen, eine Pyrrolidino- oder Tetrahydro-pyridinogruppe und

W eine Carboxy-, Aminocarbonyl-, Cyano- oder Alkoxycarbonylgruppe mit insgesamt 2 bis 4 Kohlenstoffatomen bedeuten, sowie von deren Säureadditionssalzen mit anorganischen oder organischen Säuren oder auch Basen, wenn W die Carboxygruppe darstellt, dadurch gekennzeichnet, dass

a) zur Herstellung von Verbindungen der allgemeinen Formel I, in der $R_1$ eine Aminogruppe darstellt, eine Nitroverbindung der allgemeinen Formel II

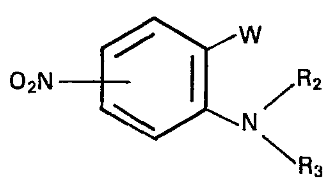

(II)

in der

$R_2$, $R_3$ und W wie eingangs definiert sind, reduziert wird oder

b) zur Herstellung von Verbindungen der allgemeinen Formel I, in der $R_1$ eine Hydroxygruppe, eine Cyanogruppe, ein Fluor-, Chlor- oder Bromatom darstellt, eine Verbindung der allgemeinen Formel Ia

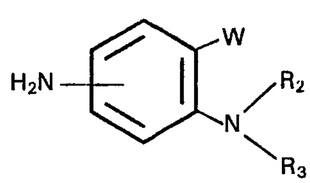

(Ia)

in der

$R_2$, $R_3$ und W wie eingangs definiert sind, mit einem Nitrit in ein Diazoniumsalz übergeführt wird und das so erhaltene Diazoniumsalz anschliessend in Gegenwart von Schwefelsäure, in Gegenwart von Kupfer oder in Gegenwart eines entsprechenden Kupfer(I)-Salzes durch Erwärmen in die entsprechende Verbindung übergeführt wird oder

c) zur Herstellung von Verbindungen der allgemeinen Formel I, in der $R_1$ eine gegebenenfalls durch eine Phenylgruppe substituierte Alkoxygruppe mit 1 bis 3 Kohlenstoffatomen oder eine Alkoxygruppe mit 1 bis 3 Kohlenstoffatomen oder eine Alkoxygruppe mit 4 bis 6 Kohlenstoffatomen darstellt, eine Hydroxyverbindung der allgemeinen Formel Ib

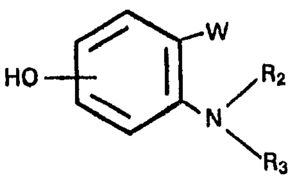

(Ib)

in der

$R_2$, $R_3$ und W wie eingangs definiert sind, mit einer Verbindung der allgemeinen Formel III

$$Z - R_1' \qquad \text{(III)}$$

in der

$R_1'$ eine gegebenenfalls durch eine Phenylgruppe substituierte Alkylgruppe mit 1 bis 3 Kohlenstoffatomen oder eine Alkylgruppe mit 4 bis 6 Kohlenstoffatomen und

Z eine nukleophile Austrittsgruppe oder zusammen mit dem benachbarten Wasserstoffatom des Restes $R_1'$ eine Diazogruppe bedeuten, alkyliert und erforderlichenfalls anschliessend hydrolisiert wird und gewünschtenfalls anschliessend eine so erhaltene Verbindung der allgemeinen Formel I, in der W eine Cyano-, Aminocarbonyl- oder Alkoxycarbonylgruppe darstellt, mittels Hydrolyse in eine entsprechende Verbindung der allgemeinen Formel I, in der W die Carboxylgruppe darstellt, übergeführt wird und/oder eine so erhaltene Verbindung der allgemeinen Formel I in ihre Säureadditionssalze mit anorganischen oder organischen Säuren oder auch Basen, wenn W die Carboxylgruppe darstellt, übergeführt wird.

2. Verfahren gemäss Anspruch 1 zur Herstellung von 2-Amino-benzoesäure-Derivaten der allgemeinen Formel I'

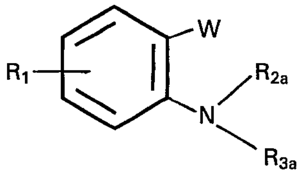

(I')

in der

$R_1$ und W wie im Anspruch 1 definiert sind,

$R_{2a}$ und $R_{3a}$ zusammen mit dem dazwischenliegenden Stickstoffatom eine N-Alkyl-cyclohexylaminogruppe, in der der Alkylteil 2 bis 4 Kohlenstoffatome enthalten kann, eine N-Alkyl-phenylamino- oder N-Alkyl-benzylaminogruppe, wobei der Alkylteil jeweils 1 bis 3 Kohlenstoffatome enthalten kann, eine cyclische Alkyleniminogruppe mit 6 bis 12 Kohlenstoffatomen, eine durch eine Alkylgruppe mit 1 bis 4 Kohlenstoffatomen, eine Alkoxygruppe mit 1 bis 3 Kohlenstoffatomen, eine Alkoxycarbonylgruppe mit insgesamt 2 bis 4 Kohlenstoffatomen oder eine Phenylgruppe substituierte Piperidinogruppe, eine durch 2, 3 oder 4 Alkylgruppen mit jeweils 1 bis 3 Kohlenstoffatomen substituierte Piperidinogruppe, eine gegebenenfalls durch eine oder zwei Alkylgruppen mit jeweils 1 bis 3 Kohlenstoffatomen substituierte Morpholino- oder Thiomorpholinogruppe, eine in 4-Stellung durch eine Alkylgruppe mit 1 bis 3 Kohlenstoffatomen, eine Alkoxycarbonylgruppe mit insgesamt 2 bis 4 Kohlenstoffatomen, eine Phenyl-, Halogenphenyl-, Pyridyl-, Benzyl- oder Fuorylgruppe substituierte Piperazinogruppe, eine gesättigte oder teilweise ungesättigte Aza-bicycloalkylgruppe mit 8 bis 10 Kohlenstoffatomen, eine 1,4-Dioxa-8-aza-spiro-alkangruppe mit insgesamt 6 bis 8 Kohlenstoffatomen, eine Pyrrolidino- oder Tetrahydro-pyridinogruppe bedeuten, sowie von deren Säureadditionssalzen mit anorganischen oder organischen Säuren oder auch Basen, wenn W die Carboxylgruppe darstellt, dadurch gekennzeichnet, dass

a) zur Herstellung von Verbindungen der allgemeinen Formel I', in der $R_1$ eine Aminogruppe darstellt, eine Nitroverbindung der allgemeinen Formel II'

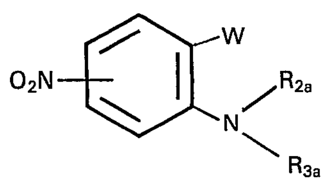

(II')

in der

$R_{2a}$, $R_{3a}$ und W wie eingangs definiert sind, reduziert wird oder

b) zur Herstellung von Verbindungen der allgemeinen Formel I', in der $R_1$ eine Hydroxygruppe, eine Cyangruppe, ein Fluor-, Chlor- oder Bromatom darstellt, eine Verbindung der allgemeinen Formel I'a

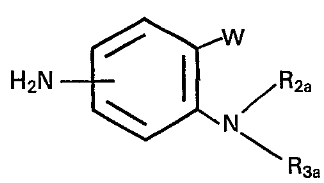

(I'a)

in der

$R_{2a}$, $R_{3a}$ und W wie eingangs definiert sind, mit einem Nitrit in ein Diazoniumsalz übergeführt wird und das so erhaltene Diazoniumsalz anschliessend in Gegenwart von Schwefelsäure, in Gegenwart von Kupfer oder in Gegenwart eines entsprechenden Kupfer(I)-Salzes durch Erwärmen in die entsprechende Verbindung übergeführt wird oder

c) zur Herstellung von Verbindungen der allgemeinen Formel I', in der $R_1$ eine gegebenenfalls

durch eine Phenylgruppe substituierte Alkoxygruppe mit 1 bis 3 Kohlenstoffatomen oder eine Alkoxygruppe mit 4 bis 6 Kohlenstoffatomen darstellt, eine Hydroxyverbindung der allgemeinen Formel I'b

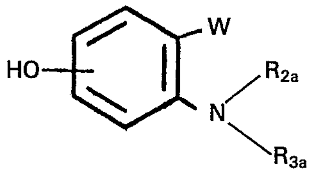
(I'b)

in der

$R_{2a}$, $R_{3a}$ und W wie eingangs definiert sind, mit einer Verbindung der allgemeinen Formel III

$$Z - R_1' \qquad (III)$$

in der

$R_1'$ und Z wie im Anspruch 1 definiert sind, alkyliert und erforderlichenfalls anschliessend hydrolysiert wird und gewünschtenfalls anschliessend eine so erhaltene Verbindung der allgemeinen Formel I', in der W eine Cyano-, Aminocarbonyl- oder Alkoxycarbonylgruppe darstellt, mittels Hydrolyse in eine entsprechende Verbindung der allgemeinen Formel I', in der W die Carboxygruppe darstellt, übergeführt wird und/oder eine so erhaltene Verbindung der allgemeinen Formel I' in ihre Säureadditionssalze mit anorganischen oder organischen Säuren oder auch Basen, wenn W die Carboxygruppe darstelltt, übergeführt wird.

3. Verfahren gemäss den Ansprüchen 1 und 2, dadurch gekennzeichnet, dass die Umsetzung in einem Lösungsmittel durchgeführt wird.

4. Verfahren gemäss den Ansprüchen 1a, 2a und 3, dadurch gekennzeichnet, dass die Reduktion bei Temperaturen zwischen 0 und 50°C durchgeführt wird.

5. Verfahren gemäss den Ansprüchen 1b, 2b und 3, dadurch gekennzeichnet, dass das Diazoniumsalz bei niedrigen Temperaturen, z.B. bei Temperaturen zwischen —10 und 5°C, hergestellt und anschliessend auf Temperaturen zwischen 15 und 90°C erhitzt wird.

6. Verfahren gemäss den Ansprüchen 1c, 2c und 3, dadurch gekennzeichnet, dass die Alkylierung bei Temperaturen zwischen 0 und 100°C durchgeführt wird.

7. Verfahren gemäss Anspruch 6, dadurch gekennzeichnet, dass die Alkylierung bei Temperaturen zwischen 15 und 70°C durchgeführt wird.

**Claims for the Contracting States:**
BE, CH, DE, FR, GB, IT, LI, LU, NL, SE

1. 2-Amino-benzoic acid derivatives of general formula I

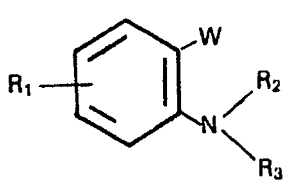
(I)

wherein

$R_1$ represents a fluorine, chlorine or bromine atom, an amino, cyano or hydroxy group, an alkoxy group with 1 to 3 carbon atoms optionally substituted by a phenyl group, or an alkoxy group with 4 to 6 carbon atoms,

$R_2$ and $R_3$ together with the nitrogen atom between them represent an N-alkylcyclohexylamino group wherein the alkyl moiety may contain 2 to 4 carbon atoms, an N-alkyl-phenylamino or N-alkyl-benzylamino group wherein the alkyl moiety may contain 1 to 3 carbon atoms, a cyclic alkyleneimino group with 6 to 12 carbon atoms, a piperidino group substituted by an alkyl group with 1 to 4 carbon atoms, an alkoxy group with 1 to 3 carbon atoms, an alkoxycarbonyl group with a total of 2 to 4 carbon atoms or a phenyl group, or a piperidino group substituted by 2, 3 or 4 alkyl groups each having 1 to 3 carbon atoms, a morpholino or thiomorpholino group optionally substituted by one or two alkyl groups each having 1 to 3 carbon atoms, a piperazino group substituted in the 4 position by an alkyl group with 1 to 3 carbon atoms, an alkoxycarbonyl group with a total of 2 to 4 carbon atoms, a phenyl, halophenyl, pyridyl, benzyl or furoyl group, a saturated or partially unsaturated azabicycloalkyl group with 8 to 10 carbon atoms, a 1,4-dioxa-8-aza-spiro-alkane group with a total of 6 to 8 carbon atoms, a pyrrolidino or tetrahydro-pyridino group and

W represents a carboxyl, aminocarboxyl, cyano or carbalkoxy group with a total of 2 to 4 carbon atoms, and the acid addition salts thereof with inorganic or organic acids or bases if W represents the carboxyl group.

2. 2-Amino-benzoic acid derivatives of general formula I as claimed in claim 1, wherein

$R_1$ represents a chlorine or bromine atom, a hydroxy, amino, cyano or benzyloxy group or an alkoxy group with 1 to 6 carbon atoms,

$R_2$ and $R_3$ together with the nitrogen atom between them represent an N-alkyl-cyclohexylamino group wherein the alkyl moiety may contain 2 to 4 carbon atoms, a cyclic alkyleneimino group with 6 to 12 carbon atoms, a piperidino group substituted in the 4 position by an alkyl group with 1 to 4 carbon atoms or by a phenyl, methoxy or carbethoxy group, a piperidino group substituted in the 3 and 5 positions by a methyl or ethyl group, a piperidino group substituted in the 3 and 5 positions by a total of 4 methyl groups, a piperazino group substituted in the 4 position by a methyl, benzyl, phenyl, chlorophenyl, pyridyl-(2), carbethoxy or fluoryl-(2) group, a morpholino or thiomorpholino group optionally substituted by one or two methyl groups, a pyrrolidino, tetrahydropyridino, N-methyl-phenylamino, N-methyl-benzylamino, 1,4-dioxa-8-aza-spiro[4,5]decan-8-yl, 1,4-dioxa-8-aza-spiro[4,6]undecan-8-yl, 3-aza-bicyclo-nonan-3-yl, tetrahydro-3-benzazepin-3-yl, decahydro-3-benzazepin-3-yl, tetrahydro-isoquinolin-2-yl, octahydro-isoquinolin-2-yl, decahydro-isoquinolin-2-yl, or octahydro-isoindol-2-yl group and

W represents a carboxyl, aminocarbonyl or cyano group,

and the acid addition salts thereof with inorganic or

organic acids or bases if W represents a carboxyl group.

3. 2-Amino-benzoic acid derivatives of general formula I as claimed in claim 1, wherein

$R_1$ represents an alkoxy group with 1 to 3 carbon atoms, a chlorine or bromine atom,

$R_2$ and $R_2$ together with the nitrogen atom between them represent a cyclic alkyleneimino group with 6 to 8 carbon atoms, a 1,4-dioxa-8-aza-spiro-[4,5]decan-8-yl, 1,4-dioxa-8-aza-spiro[4,6]undecan-8-yl, decahydro-3-benzazepino or 4-methoxy-piperidino group and

W represents a carboxyl group,

and the acid addition salts thereof with inorganic or organic acids or bases.

4. 2-Amino-benzoic acid derivatives of general formula I as claimed in claim 1, wherein $R_1$ is in the 5 position and $R_1$, $R_2$, $R_3$ and W are defined as in claim 3, and the acid addition salts thereof with inorganic or organic acids or bases.

5. 5-Chloro-2-(1,4-dioxa-8-aza-spiro[4,6]decan--8-yl)benzoic acid and the acid addition salts thereof.

6. Pharmaceutical compositions containing a compound as claimed in claims 1 to 5 together with one or more inert carriers and/or diluents.

7. Pharmaceutical compositions as claimed in claim 6 with lipid-reducing properties.

8. Process for preparing 2-amino-benzoic acid derivatives of general formula I as claimed in claims 1 to 5, and the acid addition salts thereof with inorganic or organic acids or bases if W represents a carboxyl group, characterised in that

a) in order to prepare compounds of general formula I wherein $R_1$ is an amino group, a nitro compound of general formula II

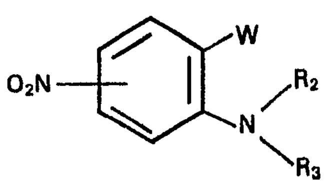

(II)

wherein $R_2$, $R_3$ and W are as hereinbefore defined, is reduced, or

b) in order to prepare compounds of general formula I wherein $R_1$ is a hydroxy group, a cyano group or a fluorine, chlorine or bromine atom, a compound of general formula Ia

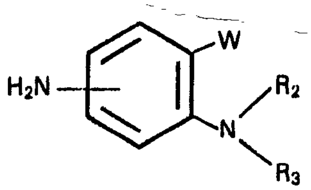

(Ia)

wherein $R_2$, $R_3$ and W are as hereinbefore defined, is converted with a nitrite into a diazonium salt and the diazonium salt thus obtained is subsequently converted into the corresponding compound by heating in the presence of sulphuric acid, copper or a corresponding copper(I)salt, or

c) in order to prepare compounds of general formula I wherein $R_1$ represents an alkoxy group with 1 to 3 carbon atoms optionally substituted by a phenyl group or an alkoxy group with 4 to 6 carbon atoms, a hydroxy compound of general formula Ib

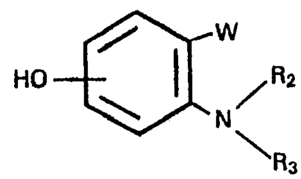

(Ib)

wherein $R_2$, $R_3$ and W are as hereinbefore defined, is alkylated with a compound of general formula III

$$Z - R_1' \qquad (III)$$

wherein

$R_1'$ represents an alkyl group with 1 to 3 carbon atoms optionally substituted by a phenyl group or an alkyl group with 4 to 6 carbon atoms and

Z represents a nucleophilic leaving group or together with the adjacent hydrogen atom of the group $R_1'$ represents a diazo group, and subsequently, if desired, hydrolysis is carried out

and subsequently, if desired, a compound of general formula I thus obtained wherein W represents a cyano, aminocarbonyl or alkoxycarbonyl group is converted by hydrolysis into a corresponding compound of general formula I wherein W represents a carboxyl group

and/or a compound of general formula I thus obtained is converted into the acid addition salts thereof with inorganic or organic acids or bases if W represents a carboxyl group.

9. Process as claimed in claim 8, characterised in that the reaction is carried out in a solvent.

10. Process as claimed in claims 8b and 9, characterised in that the diazonium salt thus prepared, e.g. the fluoroborate or the hydrosulphate is converted into the corresponding compound by heating in the presence of sulphuric acid or in the presence of a corresponding copper(I)salt such as copper/I)chloride/hydrochloric acid, copper(I)bromide/hydrobromic acid or trisodium copper(I)tetracyanide at pH 7.

**Claims for the Contracting State:** AT

1. Process for preparing 2-amino-benzoic acid derivatives of general formula I

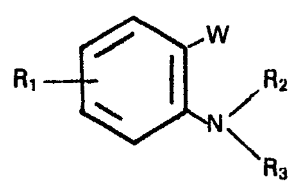

(I)

wherein

$R_1$ represents a fluorine, chlorine or bromine atom, an amino, cyano or hydroxy group, an alkoxy group with1 to 3 carbon atoms optionally substituted by a phenyl group, or an alkoxy group with 4 to 6 carbon atoms,

$R_2$ and $R_3$ together with the nitrogen atom between them represent an N-alkylcyclohexylamino group wherein the alkyl moiety may contain 2 to 4 carbon atoms, an N-alkyl-phenylamino or N-alkyl-benzylamino group wherein the alkyl moiety may contain 1 to 3 carbon atoms, a cyclic alkyleneimino group with 6 to 12 carbon atoms, a piperidino group substituted by an alkyl group with 1 to 4 carbon atoms, an alkoxy group with 1 to 3 carbon atoms, an alkoxycarbonyl group with a total of 2 to 4 carbon atoms or a phenyl group, or a piperidino group substituted by 2, 3 or 4 alkyl groups each having 1 to 3 carbon atoms, a morpholino or thiomorpholino group optionally substituted by one or two alkyl groups each having 1 to 3 carbon atoms, a piperazino group substituted in the 4 position by an alkyl group with 1 to 3 carbon atoms, an alkoxycarbonyl group with a total of 2 to 4 carbon atoms, a phenyl, halophenyl, pyridyl, benzyl or furoyl group, a saturated or partially unsaturated azabicycloalkyl group with 8 to 10 carbon atoms, a 1,4-dioxa-8-aza-spiro-alkane group with a total of 6 to 8 carbon atoms, a pyrrolidino or tetrahydro-pyridino group and

W represents a carboxyl, aminocarboxyl, cyano or carbalkoxy group with a total of 2 to 4 carbon atoms, and the acid addition salts thereof with inorganic or organic acids or bases if W represents the carboxyl group.

a) in order to prepare compounds of general formula I wherein $R_1$ is an amino group, a nitro compound of general formula II

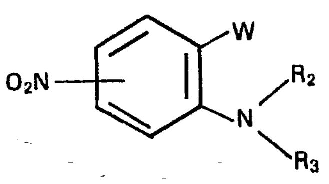

(II)

wherein $R_2$, $R_3$ and W are as hereinbefore defined, is reduced, or

b) in order to prepare compounds of general formula I wherein $R_1$ is a hydroxy group, a cyano group or a fluorine, chlorine or bromine atom, a compound of general formula Ia

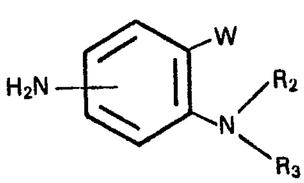

(Ia)

wherein $R_2$, $R_3$ and W are as hereinbefore defined, is converted with a nitrite into a diazonium salt and the diazonium salt thus obtained is subsequently converted into the corresponding compound by heating in the presence of sulphuric acid, copper or a corresponding copper(I)salt, or

c) in order to prepare compounds of general formula I wherein $R_1$ represents an alkoxy group with 1 to 3 carbon atoms optionally substituted by a phenyl group or an alkoxy group with 4 to 6 carbon atoms, a hydroxy compound of general formula Ib

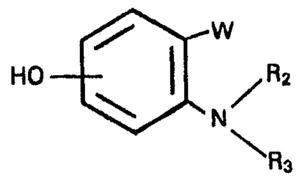

(Ib)

wherein $R_2$, $R_3$ and W are as hereinbefore defined, is alkylated with a compound of general formula III

$$Z - R_1' \qquad (III)$$

wherein

$R_1'$ represents an alkyl group with 1 to 3 carbon atoms optionally substituted by a phenyl group or an alkyl group with 4 to 6 carbon atoms and

Z represents a nucleophilic leaving group or together with the adjacent hydrogen atom of the group $R_1'$ represents a diazo group, and subsequently, if desired, hydrolysis is carried out

and subsequently, if desired, a compound of general formula I thus obtained wherein W represents a cyano, aminocarbonyl or alkoxycarbonyl group is converted by hydrolysis into a corresponding compound of general formula I wherein W represents a carboxyl group

and/or a compound of general formula I thus obtained is converted into the acid addition salts thereof with inorganic or organic acids or bases if W represents a carboxyl group.

2. Process as claimed in claim 1 for preparing 2-aminobenzoic acid derivatives of general formula I'

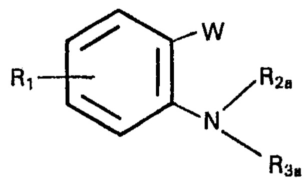

(I')

wherein

$R_1$ and W are defined as in claim 1,

$R_{2a}$ and $R_{3a}$ together with the nitrogen atom between them represent an N-alkyl-cyclohexylamino group wherein the alkyl moiety may contain 2 to 4 carbon atoms, an N-alkyl-phenylamino or N-alkyl-benzylamino group wherein the alkyl moiety may contain 1 to 3 carbon atoms, a cyclic alkyleneimino group with 6 to 12 carbon atoms, a piperidino group substituted by an alkyl group with 1 to 4 carbon atoms, an alkoxy group with 1 to 3 carbon atoms, an alkoxycarbonyl group with a total of 2 to 4 carbon atoms or a phenyl group, a piperidino group substituted by 2, 3 or 4 alkyl groups each with 1 to 3 carbon atoms, or morpholino or thiomorpholino group optionally substituted by one or two alkyl groups each with 1 to 3 carbon atoms, a piperazino group substituted in the 4 position by an alkyl group with 1 to 3 carbon atoms, an alkoxycarbonyl group with a total of 2 to 4 carbon atoms, a phenyl, halophenyl, pyridyl,

benzyl or furoyl group, a saturated or partially unsaturated aza-bicycloalkyl group with 8 to 10 carbon atoms, a 1,4-dioxa-8-aza-spiro-alkane group with a total of 6 to 8 carbon atoms, a pyrrolidino or tetrahydro-pyridino group and the acid addition salts thereof with inorganic or organic acids or bases if W represents a carboxy group, characterised in that

a) in order to prepare compounds of general formula I' wherein $R_1$ represents an amino group, a nitro compound of general formula II'

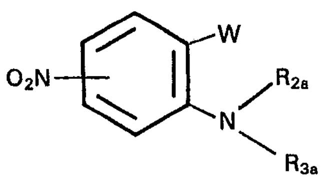

(II')

wherein

$R_{2a}$, $R_{3a}$ and W are as hereinbefore defined is reduced or

b) in order to prepare compounds of general formula I' wherein $R_1$ represents a hydroxy group, a cyano group or a fluorine, chlorine or bromine atom, a compound of general formula I'a

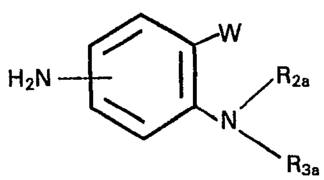

(I'a)

wherein $R_{2a}$, $R_{3a}$ and W are as hereinbefore defined, is converted with a nitrite into a diazonium salt and the diazonium salt thus obtained is subsequently converted into the corresponding compound in the presence of sulphuric acid, in the presence of copper or in the presence of a corresponding copper(I)salt, by heating, or

c) in order to prepare compounds of general formula I' wherein $R_1$ represents an alkoxy group with 1 to 3 carbon atoms optionally substituted by a phenyl group or an alkoxy group with 4 to 6 carbon atoms, a hydroxy compound of general formula I'b

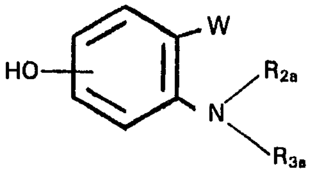

(I'b)

wherein

$R_{2a}$, $R_{3a}$ and W are as hereinbefore defined, is alkylated with a compound of general formula III

$$Z - R_1' \qquad (III)$$

wherein

$R_1'$ and Z are defined as in claim 1, and subsequently, if desired, hydrolysis is carried out and subsequently, if desired, a compound of general formula I' thus obtained wherein W represents a cyano, aminocarbonyl or alkoxycarbonyl group, is converted by hydrolysis into a corresponding compound of general formula I' wherein W represents a carboxy group,

and/or a compound of general formula I' thus obtained is converted into the acid addition salts thereof with inorganic or organic acids or bases if W represents a carboxy group.

3. Process as claimed in claims 1 and 2, characterised in that the reaction is carried out in a solvent.

4. Process as claimed in claims 1a, 2a and 3, characterised in that the reduction is carried out at temperatures of between 0 and 50°C.

5. Process as claimed in claims 1b, 2b and 3, characterised in that the diazonium salt is prepared at low temperatures, e.g. at temperatures of between —10 and 5°C, and is subsequently heated to temperatures of between 15 and 90°C.

6. Process as claimed in claims 1c, 2c and 3, characterised in that the alkylation is carried out at temperatures of between 0 and 100°C.

7. Process as claimed in claim 6, characterised in that the alkylation is carried out at temperatures of between 15 and 70°C.

**Revendications pour les Etats Contractants:**
BE, CH, DE, FR, GB, IT, LI, LU, NL, SE

1. Dérivés d'acides 2-amino-benzoïques de formule générale I:

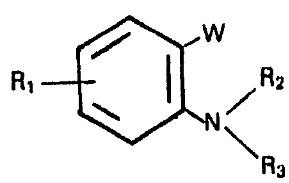

(I)

dans laquelle:

$R_1$ représente un atome de fluor, de chlore ou de brome, un groupe amino, cyano ou hydroxy, un groupe alcoxy avec 1 à 3 atomes de carbone éventuellement substitué par un groupe phényle, ou représente un groupe alcoxy avec 4 à 6 atomes de carbone,

$R_2$ et $R_3$, ensemble avec l'atome d'azote intermédiaire, représentent un groupe N-alcoyl-cyclohexylamino, dans lequel la partie alcoyle peut contenir 2 à 4 atomes de carbone, ou représentent un groupe N-alcoyl-phénylamino ou N-alcoyl-benzylamino, la partie alcoyle pouvant dans chaque cas contenir 1 à 3 atomes de carbone, ou représentent un groupe alcoylèneimino cyclique avec 6 à 12 atomes de carbone, ou représentent un groupe pipéridino substitué par un groupe alcoyle avec 1 à 4 atomes de carbone, un groupe alcoxy avec 1 à 3 atomes de carbone, un groupe alcoxycarbonyle avec en tout 2 à 4 atomes de carbone, ou représentent un groupe pipéridino substitué par deux, trois ou quatre groupes alcoyle avec chacun 1 à 3 atomes de carbone, ou représentent un groupe morpholino ou thiomorpholino éventuellement substitué par un ou deux groupes alcoyle avec

chacun 1 à 3 atomes de carbone, ou représentent un groupe pipérazino substitué en position 4 par un groupe alcoyle avec 1 à 3 atomes de carbone, un groupe alcoxycarbonyle avec en tout 2 à 4 atomes de carbone, un groupe phényle, halogénophényle, pyridyle, benzyle ou furoyle, ou représentent un groupe aza-bicycloalcoyle saturé ou en partie insaturé avec 8 à 10 atomes de carbone, ou représentent un groupe 1,4-dioxa-8-aza-spiro-alcane avec en tout 6 à 8 atomes de carbone, ou représentent un groupe pyrrolidino ou tétrahydro-pyridino, et

W représente un groupe carboxyle, aminocarbonyle, cyano ou carbalcoxy avec en tout 2 à 4 atomes de carbone, et leurs sels d'addition d'acides avec des acides minéraux ou organiques ou également des bases, lorsque W représente le groupe carboxyle.

2. Dérivés d'acides 2-amino-benzoïques de formule générale I selon la revendication 1, dans laquelle:

$R_1$ représente un atome de chlore ou de brome, un groupe hydroxy, amino, cyano ou benzyloxy ou un groupe alcoxy avec 1 à 6 atomes de carbone,

$R_2$ et $R_3$, ensemble avec l'atome d'azote intermédiaire, représentent un groupe N-alcoyl-cyclohexylamino, la partie alcoyle pouvant contenir 2 à 4 atomes de carbone, ou représentent un groupe alcoylèneimino cyclique avec 6 à 12 atomes de carbone, un groupe pipéridino substitué en position 4 par un groupe alcoyle avec 1 à 4 atomes de carbone, un groupe phényle, méthoxy ou carbéthoxy, ou représentent un groupe pipéridino substitué en positions 3 et 5 à chaque fois par un groupe méthyle ou éthyle, ou représentent un groupe pipéridino substitué en positions 3 et 5 par en tout 4 groupes méthyle, ou représentent un groupe pipérazino substitué en position 4 par un groupe méthyle, benzyle, phényle, chlorophényle, pyridyle-(2), carbéthoxy ou furoyle-(2), ou représentent un groupe morpholino ou thiomorpholino éventuellement substitué par un ou deux groupes méthyle, ou représentent un groupe pyrrolidino, tétrahydro-pyridino, N-méthyl-phénylamino, N-méthyl-benzylamino, 1,4-dioxa-8-aza-spiro[4,5]décane-8-yle, 1,4-dioxa-8-aza-spiro[4,6]undécane-8--yle, 3-aza-bicyclo-nonane-3-yle, tétrahydro-3-benzazépine-3-yle, décahydro-3-benzazépine-3-yle, tétrahydro-isoquinoléine-2-yle, octahydro-isoquinoléine-2-yle, décahydro-isoquinoléine-2-yle ou octahydro-isoindol-2-yle et

W représente le groupe carboxyle, aminocarbonyle ou cyano,

et leurs sels d'addition d'acides avec des acides minéraux ou organiques ou également des bases, lorsque W représente le groupe carbonyle.

3. Dérivés d'acides 2-amino-benzoïques de formule générale I selon la revendication 1, dans laquelle:

$R_1$ représente un groupe alcoxy avec 1 à 3 atomes de carbone, un atome de chlore ou de brome,

$R_2$ et $R_3$, ensemble avec l'atome d'azote intermédiaire, représentent un groupe alcoylèneimino cyclique avec 6 à 8 atomes de carbone, un groupe 1,4--dioxa-8-aza-spiro[4,5]décane-8-yle, 1,4-dioxa-8--aza-spiro[4,6]undécane-8-yle, décahydro-3-benzazépino ou 4-méthoxy-pipéridino et

W représente le groupe carboxyle, et leurs sels d'addition d'acides avec des acides minéraux ou organiques ou des bases.

4. Dérivés d'acides 2-amino-benzoïques de formule générale I selon la revendication 1, dans laquelle $R_1$ est en position 5 et $R_1$, $R_2$, $R_3$ et W sont définis comme dans la revendication 3, et leurs sels d'addition d'acides avec des acides minéraux ou organiques ou des bases.

5. L'acide 5-chloro-2-(1,4-dioxa-8-aza-spiro-[4,6]décane-8-yl)-benzoïque et ses sels d'addition d'acides.

6. Médicament contenant un composé selon les revendications 1 à 5 conjointement à un ou plusieurs excipients et/ou diluants inertes.

7. Médicament selon la revendication 6 avec des propriétés hypolipémiantes.

8. Procédé pour la préparation de dérivés d'acides 2-amino-benzoïques de formule générale I selon les revendications 1 à 5, ainsi que de leurs sels d'addition d'acides avec des acides minéraux ou organiques ou également des bases lorsque W représente le groupe carboxyle, caractérisé en ce que:

a) pour la préparation de composés de formule générale I, dans laquelle $R_1$ représente un groupe amino, on réduit un composé nitro de formule générale II:

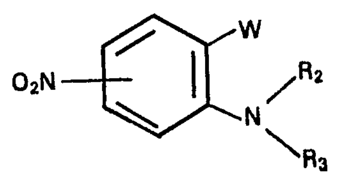

, (II)

dans laquelle:

$R_2$, $R_3$ et W sont définis comme au début, ou

b) pour la préparation de composés de formule générale I, dans laquelle $R_1$ représente un groupe hydroxy, un groupe cyano, un atome de fluor, de chlore ou de brome, on transforme un composé de formule générale Ia:

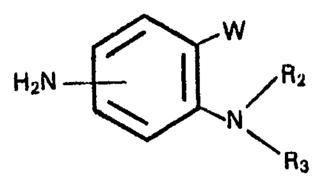

(Ia)

dans laquelle:

$R_2$, $R_3$ et W sont définis comme au début, au moyen d'un nitrite en un sel de diazonium et on transforme ensuite le sel de diazonium ainsi obtenu en présence d'acide sulfurique, en présence de cuivre ou en présence d'un sel de cuivre(I) correspondant par chauffage, en le composé correspondant ou

c) pour la préparation de composés de formule générale I, dans laquelle $R_1$ représente un groupe alcoxy avec 1 à 3 atomes de carbone éventuellement substitué par un groupe phényle ou représente un groupe alcoxy avec 4 à 6 atomes de carbone, on alcoyle un composé hydroxy de formule générale Ib:

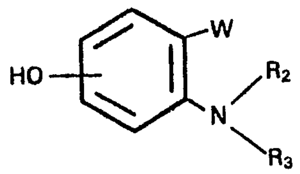

(Ib)

dans laquelle:

$R_2$, $R_3$ et W sont définis comme au début, au moyen d'un composé de formule générale III:

Z - $R_1'$ (III)

dans laquelle:

$R_1'$ représente un groupe alcoyle avec 1 à 3 atomes de carbone éventuellement substitué par un groupe phényle, ou représente un groupe alcoyle avec 4 à 6 atomes de carbone, et Z représente un groupe partant nucléophile ou représente ensemble avec l'atome d'hydrogène voisin du radical $R_1'$ un groupe diazo, et si nécessaire, on l'hydrolyse ensuite,

et si on le désire, ensuite on transforme un composé ainsi obtenu de formule générale I dans laquelle W représente un groupe cyano, aminocarbonyle ou alcoxycarbonyle au moyen d'une hydrolyse en un composé correspondant de formule générale I dans laquelle W représente le groupe carboxyle,

et/ou on transforme un composé ainsi obtenu de formule générale I en ses sels d'addition d'acides avec des acides minéraux ou organiques ou également des bases, lorsque W représente le groupe carboxyle.

9. Procédé selon la revendication 8, caractérisé en ce que la réaction est effectuée dans un solvant.

10. Procédé selon les revendications 8b et 9, caractérisé en ce que le sel de diazonium ainsi préparé, par exemple le fluoroborate, l'hydrosulfate, est transformé en le composé correspondant à pH 7, par chauffage en présence d'acide sulfurique ou en présence d'un sel de cuivre(I) correspondant tel que le chlorure de cuivre(I)/acide chlorhydrique, le bromure de cuivre(I)/acide bromhydrique ou le tétracyanure de cuivre(I) trisodé.

**Revendications pour l'Etat Contractant:** AT

1. Procédé pour la préparation de dérivés d'acides 2-amino-benzoïques de formule générale I:

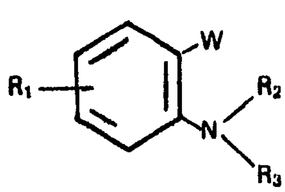

(I)

dans laquelle:

$R_1$ représente un atome de fluor, de chlore ou de brome, un groupe amino, cyano ou hydroxy, un groupe alcoxy avec 1 à 3 atomes de carbone éventuellement substitué par un groupe phényle, ou représente un groupe alcoxy avec 4 à 6 atomes de carbone,

$R_2$ et $R_3$, ensemble avec l'atome d'azote intermédiaire, représentent un groupe N-alcoyl-cyclohexylamino, dans lequel la partie alcoyle peut contenir 2 à 4 atomes de carbone, un groupe N-alcoyl-phénylamino ou N-alcoyl-benzylamino, la partie alcoyle pouvant à chaque fois contenir 1 à 3 atomes de carbone, ou représentent un groupe alcoylèneimino cyclique avec 6 à 12 atomes de carbone, ou représentent un groupe pipéridino substitué par un groupe alcoyle avec 1 à 4 atomes de carbone, un groupe alcoxy avec 1 à 3 atomes de carbone, un groupe alcoxycarbonyle avec en tout 2 à 4 atomes de carbone ou un groupe phényle, ou représentent un groupe pipéridino substitué par deux, trois ou quatre groupes alcoyle avec chacun 1 à 3 atomes de carbone, ou représentent un groupe morpholino ou thiomorpholino éventuellement substitué par un ou deux groupes alcoyle avec chacun 1 à 3 atomes de carbone, ou représentent un groupe pipérazino substitué en position 4 par un groupe alcoyle avec 1 à 3 atomes de carbone, un groupe alcoxycarbonyle avec en tout 2 à 4 atomes de carbone, un groupe phényle, halogénophényle, pyridyle, benzyle ou furoyle, ou représentent un groupe aza-bicycloalcoyle saturé ou en partie insaturé avec 8 à 10 atomes de carbone, un groupe 1,4-dioxa-8-aza-spiro-alcane avec en tout 6 à 8 atomes de carbone, un groupe pyrrolidino ou tétrahydro-pyridino, et

W représente un groupe carboxy, aminocarbonyle, cyano ou alcoxycarbonyle avec en tout 2 à 4 atomes de carbone, ainsi que de leurs sels d'addition d'acides avec des acides minéraux ou organiques ou également des bases lorsque W représente le groupe carboxy, caractérisé en ce que:

a) pour la préparation de composés de formule générale I, dans laquelle $R_1$ représente un groupe amino, on réduit un composé nitro de formule générale II:

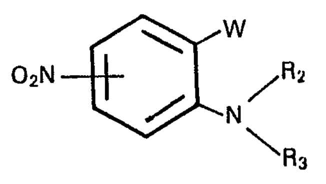

(II)

dans laquelle:

$R_2$, $R_3$ et W sont définis comme au début, ou

b) pour la préparation de composés de formule générale I, dans laquelle $R_1$ représente un groupe hydroxy, un groupe cyano, un atome de fluor, de chlore ou de brome, on transforme un composé de formule générale Ia:

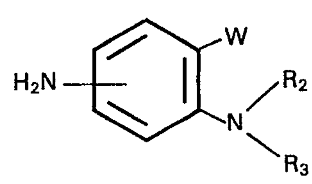

(Ia)

dans laquelle:

$R_2$, $R_3$ et W sont définis comme au début, au

moyen d'un nitrite en un sel de diazonium et on transforme ensuite le sel de diazonium ainsi obtenu en présence d'acide sulfurique, en présence de cuivre ou en présence d'un sel de cuivre(I) correspondant par chauffage, en le composé correspondant ou

c) pour la préparation de composés de formule générale I, dans laquelle $R_1$ représente un groupe alcoxy avec 1 à 3 atomes de carbone éventuellement substitué par un groupe phényle ou représente un groupe alcoxy avec 4 à 6 atomes de carbone, on alcoyle un composé hydroxy de formule générale Ib:

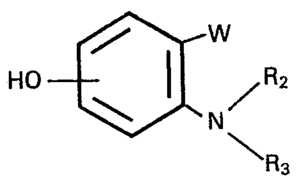

(Ib)

dans laquelle:

$R_2$, $R_3$ et W sont définis comme au début, au moyen d'un composé de formule générale III:

$$Z - R_1' \qquad (III)$$

dans laquelle:

$R_1'$ représente un groupe alcoyle avec 1 à 3 atomes de carbone éventuellement substitué par un groupe phényle, ou représente un groupe alcoyle avec 4 à 6 atomes de carbone, et

Z représente un groupe partant nucléophile ou représente ensemble avec l'atome d'hydrogène voisin du radical $R_1'$ un groupe diazo, et si nécessaire, on hydrolyse ensuite,

et si on le désire, ensuite on transforme un composé ainsi obtenu de formule générale I, dans laquelle W représente un groupe cyano, aminocarbonyle ou alcoxycarbonyle au moyen d'une hydrolyse en un composé correspondant de formule générale I, dans laquelle W représente le groupe carboxy,

et/ou on transforme un composé ainsi obtenu de formule générale I en ses sels d'addition d'acides avec des acides minéraux ou organiques ou également des bases, lorsque W représente le groupe carboxy.

2. Procédé selon la revendication 1 pour la préparation de dérivés d'acides 2-amino-benzoïques de formule générale I':

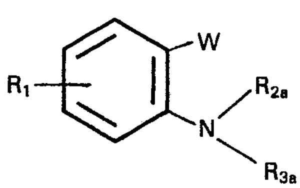

(I')

dans laquelle:

$R_1$ et W sont définis comme dans la revendication 1,

$R_{2a}$ et$R_{3a}$, ensemble avec l'atome d'azote intermédiaire, représentent un groupe N-alcoyl-cyclohexylamino, dans lequel la partie alcoyle peut contenir 2 à 4 atomes de carbone, un groupe N-alcoyl-phénylamino ou N-alcoyl-benzylamino, la partie alcoyle pouvant à chaque fois contenir 1 à 3 atomes de carbone, ou représentent un groupe alcoylèneimino cyclique avec 6 à 12 atomes de carbone, un groupe pipéridino substitué par un groupe alcoyle avec 1 à 4 atomes de carbone, un groupe alcoxy avec 1 à 3 atomes de carbone, un groupe alcoxycarbonyle avec en tout 2 à 4 atomes de carbone ou un groupe phényle, ou représentent un groupe pipéridino substitué par deux, trois ou quatre groupes alcoyle avec chacun 1 à 3 atomes de carbone, ou représentent un groupe morpholino ou thiomorpholino éventuellement substitué par un ou deux groupes alcoyle avec chacun 1 à 3 atomes de carbone, ou représentent un groupe pipérazino substitué en position 4 par un groupe alcoyle avec 1 à 3 atomes de carbone, un groupe alcoxycarbonyle avec en tout 2 à 4 atomes de carbone, un groupe phényle, halogénophényle, pyridyle, benzyle ou furoyle, ou représentent un groupe aza-bicycloalcoyle saturé ou en partie insaturé avec 8 à 10 atomes de carbone, un groupe 1,4-dioxa-8-aza-spiro-alcane avec en tout 6 à 8 atomes de carbone, un groupe pyrrolidino ou tétrahydro-pyridino, ainsi que le leurs sels d'addition d'acides avec des acides minéraux ou organiques ou également des bases lorsque W représente le groupe carboxy, caractérisé en ce que:

a) pour la préparation de composés de formule générale I', dans laquelle $R_1$ représente un groupe amino, on réduit un composé nitro de formule générale II':

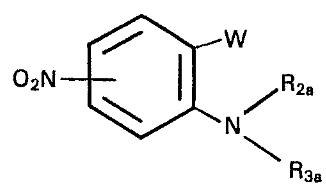

(II')

dans laquelle:

$R_{2a}$, $R_{3a}$ et W sont définis comme au début, ou

b) pour la préparation de composés de formule générale I', dans laquelle $R_1$ représente un groupe hydroxy, un groupe cyano, un atome de fluor, de chlore ou de brome, on transforme un composé de formule générale I'a:

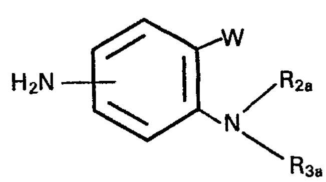

(I'a)

dans laquelle:

$R_{2a}$, $R_{3a}$ et W sont définis comme au début, au moyen d'un nitrite en un sel de diazonium et on transforme ensuite le sel de diazonium ainsi obtenu en présence d'acide sulfurique, en présence de cuivre ou en présence d'un sel de cuivre(I) correspondant par chauffage en le composé correspondant ou

c) pour la préparation de composés de formule générale I', dans laquelle $R_1$ représente un groupe alcoxy avec 1 à 3 atomes de carbone éventuellement substitué par un groupe phényle, ou représente un

groupe alcoxy avec 4 à 6 atomes de carbone, on alcoyle un composé hydroxy de formule générale I'b:

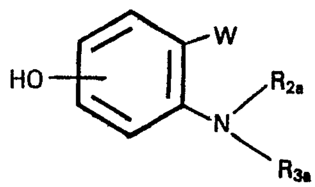

(I'b)

dans laquelle:

$R_{2a}$, $R_{3a}$ et W sont définis comme au début, au moyen dun composé de formule générale III:

$$Z - R_1' \qquad (III)$$

dans laquelle:

$R_1'$ et Z sont définis comme dans la revendication 1, et si nécessaire, on l'hydrolyse ensuite et si on le désire ensuite, on transforme un composé ainsi obtenu de formule générale I', dans laquelle W représente un groupe cyano, aminocarbonyle ou alcoxycarbonyle, au moyen d'une hydrolyse en un composé correspondant de formule générale I', dans laquelle W représente le groupe carboxy,

et/ou on transforme un composé ainsi obtenu de formule générale I' en ses sels d'addition d'acides avec des acides minéraux ou organiques ou également des bases, lorsque W représente le groupe carboxy.

3. Procédé selon les revendications 1 et 2, caractérisé en ce que la réaction est effectuée dans un solvant.

4. Procédé selon les revendications 1a, 2a et 3, caractérisé en ce que la réduction est effectuée à des températures entre 0 et 50°C.

5. Procede selon les revendications 1b, 2b et 3, caractérisé en ce que le sel de diazonium est préparé à des températures basses, par exemple à des températures entre —10 et 5°C, et ensuite chauffé à des températures entre 15 et 90°C.

6. Procédé selon les revendications 1c, 2c et 3, caractérisé en ce que l'alcoylation est effectuée à des températures entre 0 et 100°C.

7. Procédé selon la revendication 6, caractérisé en ce que l'alcoylation est effectuée à des températures entre 15 et 70°C.